# EUROPEAN PATENT SPECIFICATION

(11) **EP 2 547 766 B1**
(45) Date of publication and mention of the grant of the patent: **27.12.2017**
(21) Application number: 11731490.6
(22) Date of filing: 17.03.2011
(51) Int. Cl.: C12N 9/88, A01N 25/00, A01H 5/10, C12N 15/82

(54) **HERBICIDE-TOLERANT PLANTS**
HERBIZIDTOLERANTE PFLANZEN
PLANTES TOLÉRANTES AUX HERBICIDES

(30) Priority: 05.11.2010 US 410802 P; 17.03.2010 US 314901 P; 24.11.2010 US 417132 P
(43) Date of publication of application: 23.01.2013
(73) Proprietor: BASF Agrochemical Products B.V., 6835 EA Arnhem (NL)
(72) Inventor: VANTIEGHEM, Herve, 76297 Stutensee (DE); PFENNING, Matthias, 67365 Schwegenheim (DE); BREMER, Hagen, 67063 Ludwigshafen (DE); KEHLER, Ron, Mississauga Ontario L5R 4H1 (CA); SCHOENHAMMER, Alfons, 67117 Limburgerhof (DE)
(74) Representative: Reitstötter Kinzebach
(86) International application number: PCT/IB2011/000704
(87) International publication number: WO 2011/114232

(56) References cited:
- WO-A1-2009/046334
- WO-A1-2010/037057
- US-A- 5 434 283
- US-B1- 6 214 769
- RAY K ET AL: "Mutant acetolactate synthase gene is an efficient in vitro selectable marker for the genetic transformation of Brassica juncea (oilseed mustard)", JOURNAL OF PLANT PHYSIOLOGY, FISCHER, STUTTGART, DE, vol. 161, no. 9, 20 September 2004 (2004-09-20), pages 1079-1083, XP004955445, ISSN: 0176-1617, DOI: 10.1016/J.JPLPH.2004.02.001

## Description

### CROSS-REFERENCE TO RELATED APPLICATIONS

This application claims priority to U.S. Provisional Applications for Patent Serial Nos. 61/314,901 filed March 17,2010; 61/410,802 filed November 5, 2010; and 61/417,132 filed November 24, 2010.

### BACKGROUND OF THE INVENTION

Acetohydroxyacid synthase (AHAS; EC 4.1.3.18) is the first enzyme that catalyzes the biochemical synthesis of the branched chain amino acids valine, leucine, and isoleucine (Singh B. K., 1999 Biosynthesis of valine, leucine, and isoleucine in: Singh B. K. (Ed) Plant amino acids. Marcel Dekker Inc. New York, N.Y. Pg 227-247). AHAS is the site of action of four structurally diverse herbicide families including the sulfonylureas (LaRossa R A and Falco S C, 1984 Trends Biotechnol. 2:158-161), the imidazolinones (Shaner et al., 1984 Plant Physiol. 76:545-546), the triazolopyrimidines (Subramanian and Gerwick, 1989 Inhibition of acetolactate synthase by triazolopyrimidines in (ed) Whitaker J R, Sonnet P E Biocatalysis in agricultural biotechnology. ACS Symposium Series, American Chemical Society. Washington, D.C. Pg 277-288), and the pyrimidinylbenzoates (Subramanian et al., 1990 Plant Physiol 94:239-244.). Imidazolinone (IMI) and sulfonylurea (SU) herbicides are widely used in modern agriculture due to their effectiveness at very low application rates and relative non-toxicity in animals. By inhibiting AHAS activity, these families of herbicides prevent further growth and development of susceptible plants including many weed species.

Imidazolinone-tolerant canola has been developed through mutagenesis and selection with imidazolinone herbicides (S. Tan et al, Pest Management Science 61, 2005, 246). Commercial varieties were developed on the basis of the two most tolerant mutants, PM1 and PM2, and are currently marketed under the Clearfield® trademark. PM1 is known to be tolerant to imidazolinones only, whereas PM2 is cross-tolerant to both imidazolinones and sulfonylureas.

Although a PM2 mutant gene can provide some level of tolerance to imidazolinone and/or sulfonylurea herbicides, those oilseed rape (OSR) plants reported to date that contain a single PM2 mutant gene have exhibited insufficient tolerance to SU herbicides. For example, thifensulfuron application to the PM-mutant gene-containing, spring-type *B. napus* cultivar, 45A77, was shown to lead to reduced canola biomass, herbicide injury symptoms, or delayed maturity (R. Degenhardt et al., Weed Technology 19, 2005, 608).

In addition, there are four commercially available winter oilseed rape (WOSR) lines that are recognized in the art to provide tolerance to soil residues of sulfonylurea herbicides that are present as carry-over from prior wheat or pea crops. These four are *B. napus* variety 'Sumner' from Kansas State University; Roundup Ready lines DKW46-15 and DKW47-15 from DeKalb; and Roundup Ready line HyClass 115W from Croplan Genetics.

WO 2009/046334 describes mutated *Brassica* AHAS genes. A mutant *B. juncea* AHAS gene is described in Ray et al. (J Plant Physiol 2004, 161(9), 1079-1083). US 5,434,283 A describes deposits of rape plants which are tolerant to imidazolinone herbicides or to both chlorsulfuron and imidazolinone herbicides.

However, carry-over tolerant WOSR plants have been found to exhibit limited tolerance to soil SU herbicide residues present above carryover levels, i.e., above a residual concentration of about 0.5x, remaining from the applied herbicide dose. Thus, there remains a need in the art for winter-type *Brassica* plants that exhibit tolerance to greater than carryover levels of sulfonylurea herbicide.

### BRIEF SUMMARY OF THE INVENTION

The present invention provides methods employing herbicide-tolerant (HT) winter-type *Brassica* plants expressing one or more herbicide-tolerant AHASL genes as defined in the claims. HT winter-type *Brassica* plants described herein, containing one or fewer AHASL gene encoding a PM2 or similar mutations, unexpectedly exhibit no significant injury when contacted with an amount of SU herbicide that typically causes a non-tolerant plant to exhibit significant injury. For example, when contacted with a 1x rate of SU herbicide, on a scale from 1 to 10, with 1 indicating no visible damage and 10 indicating death of the plant, an HT *Brassica* plant hereof exhibits a score of 1.

The present invention is based on an unexpected discovery that winter-type *Brassica* crops (as exemplified by winter oilseed rape (WOSR), i.e., winter-type *B. napus* canola) that contains a mutant AHAS gene(s) providing imidazolinone tolerance, or that contain a mutant AHAS gene that normally provides insufficient SU herbicide tolerance, e.g., in spring-type OSR, surprisingly exhibit a commercially useful level of tolerance to certain sulfonylurea herbicides, i.e., a subgroup of the sulfonylurea herbicides. Yet, when the same genes are present in spring-types of the same *Brassica* crops, even if they do provide a commercially useful level of imidazolinone herbicide tolerance, the crops are found to be susceptible to these commercial levels of sulfonylurea herbicides.

The surprisingly high level of SU-herbicide tolerance exhibited by the winter-type *Brassica* plants described herein can occur when an herbicide-tolerant AHASL gene of interest is present in the *Brassica* A genome, preferably where such HT-AHASL gene is a variant of and is located at the plant's A genome native AHASL locus. Such an HT-AHASL can be one obtained by mutagenesis, such as random mutagenesis of a *Brassica* A genome AHASL. When the herbicide-tolerant AHASL gene of interest is present solely in a genome other than the *Brassica* A genome, winter-type *Brassica* plants are found to be susceptible to the commercial levels of such SU-herbicides. Spring-types of the same *Brassica* crops having the herbicide-tolerant AHASL gene of interest in the *Brassica* A genome are also found to be susceptible to such levels of SU-herbicides.

HT winter-type *Brassica* plants employed in methods of the present invention are *Brassica* plants having at least one herbicide tolerant AHASL (HT-AHASL) gene, wherein only one of the HT-AHASL genes in the plant encodes a sulfonylurea herbicide tolerance (SU-HT) mutation that is W574X and is a mono-SU-HT-AHASL gene, which can optionally encode Other HT mutation(s), and wherein said mono-SU-HT-AHASL gene is located in the A genome of said *Brassica* plant. A further SU-HT mutation described herein is P197X. Winter-type *Brassica* plants employed in methods of the present invention having such a mono-SU-HT-AHASL gene can further contain in any genome thereof a second HT-AHASL gene encoding no P197X or W574X substitutions, but encoding a different HT substitution, such as an Other HT mutation. For example, winter-type *Brassica* plants having an HT-AHASL gene encoding W574L homozygously, hemizygously or heterozygously in the A genome can also have a second HT-AHASL gene in the *Brassica* C genome, e.g., an AHAS gene encoding a S653N mutation.

The present invention provides methods of employing such HT winter-type *Brassica* plants including methods for protecting a winter-type *Brassica* crop from weeds, methods for selecting HT winter-type *Brassica* plants, and methods for providing yield protection for a winter-type *Brassica* crop. These methods can include performing post-emergent treatment or pre-emergent herbicide treatment of the HT winter-type *Brassica* plants.

### BRIEF DESCRIPTION OF THE DRAWINGS

Fig. 1 provides a partial nucleotide sequence (SEQ ID NO:1) of a *B. napus* AHASL gene encoding the PM2 mutation (BnAHASL1A_PM2).
Fig. 2 provides a partial nucleotide sequence (SEQ ID NO:2) of a *B. napus* AHASL encoding the PM1 mutation (BnAHASL1C_PM1).
Fig. 3 provides a partial amino acid sequence (SEQ ID NO:3) of a *B*. *napus* AHASL gene having the PM2 mutation (BnAHASL1A_PM2).
Fig. 4 provides a partial amino acid sequence (SEQ ID NO:4) *of B. napus* AHASL having the PM1 mutation (BnAHASL1C_PM1).
Fig. 5 provides a second nucleotide sequence (SEQ ID NO:5) of a *Brassica* AHASL gene encoding the PM2 mutation (AHASL1A_PM2).
Fig. 6 provides a second amino acid sequence (SEQ ID NO:6) of a *Brassica* AHASL having the PM2 mutation (AHASL1A_PM2).
Fig. 7 is a graph showing AHAS enzyme activity in the presence of an imidazolinone herbicide.
Fig. 8 is a graph showing AHAS enzyme activity in the presence of a sulfonylurea herbicide.

### DETAILED DESCRIPTION OF THE INVENTION

### Definitions

As used herein, standard one letter abbreviations for amino acids will be used, for example, A indicates alanine, P indicates proline, W indicates tryptophan, X indicates any amino acid, etc. Mutations as compared to the wild-type sequence will be indicated by specifying the wild-type amino acid and position followed by the amino acid present in the mutant. For example, P197X will be used to indicate that the proline at position 197 can be substituted with any amino acid.

As used herein, the amino acid positions refer to the polypeptide of the large subunit of the plastidic, *Brassica* AHAS enzymes (AHASL). Amino acid positions in a *Brassica* AIIASL referred to herein are numbered according to the industry standard numbering of residues corresponding to those in the *Arabidopsis thaliana (At)* AHASL sequence, and can be denoted with an (*At*). For example, P197(*At*) refers to the proline residue at the position in a *Brassica* AHASL that corresponds to the proline at position 197 of the *Arabidopsis thaliana* AHASL.

As used herein, "tolerant" or "herbicide-tolerant" indicates a plant or portion thereof capable of growing in the presence of an amount of herbicide that normally causes growth inhibition in a non-tolerant (e.g., a wild-type) plant or portion thereof. Levels of herbicide that normally inhibit growth of a non-tolerant plant are known and readily determined by those skilled in the art. Examples include the amounts recommended by manufacturers for application. The maximum rate is an example of an amount of herbicide that would normally inhibit growth of a non-tolerant plant.

As used herein, "herbicide tolerant (HT) AHASL" refers to the AHASL polypeptide expressed from one HT AHASL allele of an AHASL gene in a plant cell and/or from either or both of two homologous alleles of the same HT AHASL gene, i.e., in the same genome of the plant cell, whereby the HT-AHASL can provide herbicide tolerance to an AHAS enzyme of the plant cell. An HT-AHASL gene can be recombinant, or can be obtained by application of a mutagenesis process, a breeding process, or other process known in the art. Such a gene can be hemizygous, heterozygous, or homozygous.

As used herein, "AHAS" and "AHASL" respectively refer to functional, plastidic AHAS enzymes and AHASL polypeptides thereof, i.e., which are functional in cells of the *Brassica* plants as described herein. Similarly, terms such as "gene" and "polynucleotide", when used in reference to those encoding such an "AHAS" and "AHASL," refer to functional genes therefor, i.e., genes that are expressible in such a cell.

As used herein in regard to herbicides useful in various embodiments hereof, terms such as AHAS inhibitor, ACCase inhibitor, PPO inhibitor, EPSPS inhibitor, imidazolinone, sulfonylurea, and the like, refer to those agronomically acceptable herbicide active ingredients (A.I.) recognized in the art. Similarly, terms such as fungicide, nematicide, pesticide, and the like, refer to other agronomically acceptable active ingredients recognized in the art.

When used in reference to a particular mutant enzyme or mutant polypeptide thereof, terms such as herbicide tolerant (HT) and herbicide tolerance refer to the ability of such enzyme (or the ability of the polypeptide to confer to its enzyme the ability) to tolerate an herbicide A.I. that would normally inactivate or inhibit the activity of the wild-type (non-mutant) version of said enzyme. When used specifically in regard to an AHAS enzyme, or AHASL polypeptide, it refers specifically to the ability to tolerate an AHAS-inhibitor. Classes of AHAS-inhibitors include sulfonylureas, imidazolinones, triazolopyrimidines, sulfonylaminocarbonyltriazolinones, and pyrimidinylbenzoates.

As used herein, "recombinant" refers to an organism having genetic material from different sources as a result of human application of a recombinogenic technique.

As used herein, "mutagenized" refers to an organism having an altered genetic material as compared to the genetic material of a corresponding wild-type organism, wherein the alteration(s) in genetic material were induced and/or selected by human action. Examples of human action that can be used to produce a mutagenized organism include, but are not limited to, tissue culture of plant cells (e.g., calli) in sub-lethal concentrations of herbicides (e.g., sulfonylurea herbicides), treatment of plant cells with a chemical mutagen and subsequent selection with herbicides (e.g., sulfonylurea herbicides); or by treatment of plant cells with x-rays and subsequent selection with herbicides (e.g., sulfonylurea herbicides). Any method known in the art can be used to induce mutations. Methods of inducing mutations can induce mutations in random positions in the genetic material or can induce mutations in specific locations in the genetic material (i.e., can be directed mutagenesis techniques).

As used herein, a "genetically modified organism" (GMO) is an organism whose genetic characteristics have been altered by human effort causing insertion of genetic material from another source organism or progeny thereof that retain the inserted genetic material. The source organism can be of a different type of organism (e.g., a GMO plant can contain bacterial genetic material) or from the same type of organism (e.g., a GMO plant can contain genetic material from another plant). As used herein, recombinant and GMO are considered synonyms and indicate the presence of genetic material from a different source whereas mutagenized indicates altered genetic material from a corresponding wild-type organism but no genetic material from another source organism.

As used herein, "wild-type" or "corresponding wild-type plant" means the typical form of an organism or its genetic material, as it normally occurs, as distinguished from, e.g., mutagenized and/or recombinant forms.

As used herein, an herbicide-tolerance-inducing mutation "HT-mutation" is an alteration in the amino acid sequence of an AHASL enzyme that confers tolerance to one or more herbicides (i.e., sulfonylurea herbicides, imidazolinone herbicides, etc).

An HT-mutation can be an "SU-HT-mutation", i.e., a mutation selected from the group consisting of P197X and W574X. For example, an SU-HT-mutation can be selected from the group consisting of P197S, P197A, P197E, P197L, P197Q, P197R, P197S, P197V, P197W, P197Y, P197I, P197H, P197C, and P197G. In some embodiments, an SU-HT-mutation can be selected from the group consisting of W574L, W574M, W574C, W574S, W574R, W574G, W574A, W574F, W574Q, and W574Y. In some embodiments, an SU-HT-mutation can comprise W574L.

In some embodiments, an HT-mutation can be an "Other HT-mutation". As used herein, an "Other HT-mutation" is an alteration in the amino acid sequence of an AHASL enzyme that confers tolerance to one or more herbicides (i.e., sulfonylurea herbicides, imidazolinone herbicides, etc) wherein the alteration is at a position other than proline 197 or tryptophan 574. The following Table 1 provides a list of possible sites for Other HT-mutations, permissible substitutions, preferred substitutions, and more preferred substitutions. X indicates any amino acid.

**Table 1. "Other" HT Mutations**

| **w/t (*At*)** | **Permissible Sub.** | **Pref. Sub.** | **More. Pref.** |
|---|---|---|---|
| G121 | X | NSA D | |
| A122 | X | TV DPY (**or** X) | TV |
| M124 | X | E I | |
| R142 | X | K | |
| V196 | X | M | |
| R199 | X | AE | AE |
| T203 | X | I | |
| A205 | X | V CDERTWYN | V |
| F206 | X | RAHWY | |
| K256 | X | DENPTG | |
| M351 | X | CKVGPQY | |
| H352 | X | FMQ | |
| R373 | X | F | |
| D375 | X | NAE | |
| D376 | X | EVN GPSWAC | |
| R377 | X | K | |
| M570 | X | ANC | |
| V571 | X | ACNYIQSW | |
| F578 | X | CGLNRDEIKPSW | |
| S653 | X | N IFT | N |
| G654 | X | QCED | E |

In some embodiments, Other HT-mutations can be selected from the group consisting of A122X, R199X, A205X, S653X, and G654X, and combinations thereof. In other embodiments, Other HT-mutations can be selected from the group consisting of A122T, A122V, A122D, A122P, A122Y, R199A, R199E, A205V, A205C, A205D, A205E, A205R, A205T, A205W, A205Y, A205N, S653N, S653I, S653F, S653T, G654Q, G654C, G654E, G654D, and combinations thereof. In some embodiments, Other HT-mutations can be selected from the group consisting of A122T, A122V, R199A, R199E, A205V, S653N, G654E, and combinations thereof.

### Plants

Sources of useful plastidic AHASL genes can be provided from any of the following deposited cell lines listed in Table 2, of *Brassica napus* (Bn) and *Brassica juncea* (Bj), wherein their AHAS-inhibitor-tolerant (HT) AHAS large subunit (AHASL) alleles are referred to as shown below, with the final letter indicating the *Brassica* genome (A, B, or C) to which the allele is native: BnAHASL1A or BnAHASL1C for *B. napus,* and BjAHASL1A or BjAHASL1B for *B. juncea.* Note that AHASL mutation positions are stated with reference to the standardized nomenclature in the field, in which the *Arabidopsis thaliana* (*At*) plastidic AHASL polypeptide provides the standard for residue position numbering.

**Table 2. Examples of Brassica Line Sources for Useful AHASL Genes**

| US Patent No. 5,545,821 to Wong et al. | | | | | | | |
|---|---|---|---|---|---|---|---|
| **Name of Line** | | **Species** | | **ATCC Deposit** | | **AHAS Allele** | **Mutation** |
| PM-1 | | *B. napus* | | 40683 | | BnAHASL1C | S653(*At*)N |
| PM-2 | | *B. napus* | | 40684 | | BnAHASL1A | W574(*At*)L |
| | | | | | | | |

| PCT Application No. PCT/US09/58169 to Beetham et al. | | | | | | | |
|---|---|---|---|---|---|---|---|
| **Name of Line** | | **Species** | | **ATCC Deposit** | | **AHAS Allele(s)** | **Mutation(s)** |
| BnCL120C7 | | *B. napus* | | PTA-9278 | | BnAHASL1A | A122(*At*)T |
| BnCL131A1 | | *B. napus* | | PTA-9279 | | BnAHASL1A | A122(*At*)T + S653(*At*)N |
| BnCL140B3 | | *B. napus* | | PTA-9402 | | BnAHASL1A | A122(*At*)T + S653(*At*)N |
| BnCL140C7 | | *B. napus* | | PTA-9403 | | BnAHASL1A | A122(*At*)T + S653(*At*)N |
| PM1PM2/CL131A1 | | *B. napus* | | PTA-10321 | | BnAHASL1C | S653(*At*)N |
| | | | | | | BnAHASL1A | W574(*At*)L |
| | | | | | | BnAHASL1A | A122(*At*)T + S653(*At*)N |
| | | | | | | | |

| US Patent No. 7,355,098 to Yao et al. | | | | | | | |
|---|---|---|---|---|---|---|---|
| **Name of Line** | | **Species** | | **ATCC Deposit** | | **AHAS Allele** | **Mutation** |
| J04E-0044 | | *B. juncea* | | PTA-6324 | | BjAHASL1B | S653(*At*)N |
| J04E-0122 | | *B. juncea* | | PTA-7944 | | BjAHASL1A | A122(*At*)T |
| J04E-0130 | | *B. juncea* | | PTA-7945 | | BjAHASL1B | A122(*At*)T |
| J04E-0139 | | *B. juncea* | | PTA-7946 | | BjAHASL1A | S653(*At*)N |
| | | | | | | | |

| PCT Publication WO 2009/031031 to Yao et al. | | | | | | | |
|---|---|---|---|---|---|---|---|
| **Name of Line** | **Species** | | **ATCC Deposit** | | **AHAS Allele(s)** | | **Mutation(s)** |
| J05Z-07801 | *B. juncea* | | PTA-8305 | | BjAHASL1B | | S653(*At*)N |
| | | | | | BnAHASL1A | | W574(*At*)L |

Although exemplified with reference to winter-type, AHAS-inhibitor-tolerant *Brassica napus* Canola/OSR varieties, it is believed that in various embodiments, the presently described methods using sulfonylurea herbicides can be employed with other commercially valuable, winter-type, AHAS-inhibitor-tolerant *Brassica* species, such as *B. oleracea, B. rapa, B. nigra*, and *B. juncea.* AHAS-inhibitor-tolerant *Brassica* lines described as useful herein can be employed in the weed control methods either directly or indirectly, i.e., either as crops for herbicide treatment or as AHAS-inhibitor-tolerance trait donor lines for development, as by traditional plant breeding, to produce other winter-type *Brassica* varietal and/or hybrid crops containing such trait or traits. All such resulting variety or hybrids crops, containing the ancestral AHAS-inhibitor-tolerance trait or traits can be referred to herein as progeny of the ancestral, AHAS-inhibitor-tolerant line(s). In the case of *Brassica* A-, B-, and C-genome AHASL traits, these can be bred into winter-type *Brassica* species having a corresponding genome, e.g.: *B. napus* (AACC), *B. juncea.*(*AABB*), *B. oleracea* (CC), *B. rapa* (AA), *B. nigra* (BB), *B. carinata* (BBCC), and *Raphanobrassica* varieties that are progeny of a cross between any of the foregoing and a *Raphanus* spp., e.g., *Raphanobrassica* var. 'rabbage' (RRCC) from *B. oleracea* x *Raphanus sativus* or *Raphanobrassica* var. 'raparadish' (RRAA) from *B. rapa* x *Raphanus sativus.* Among these, *B. napus, B. rapa,* and *B. juncea* are of particular interest, with *B. napus* being preferred in some embodiments.

Plants employed in methods of the invention can include those plants which, in addition to having been rendered sulfonylurea-tolerant, have been subjected to further genetic modifications by breeding, mutagenesis or genetic engineering, e.g. have been rendered tolerant to applications of specific other classes of herbicides, such as auxin herbicides, dicamba or 2,4-D; bleacher herbicides such as hydroxyphenylpyruvate dioxygenase (HPPD) inhibitors or phytoene desaturase (PDS) inhibitors; enolpyruvyl shikimate 3-phosphate synthase (EPSP) inhibitors such as glyphosate; glutamine synthetase (GS) inhibitors such as glufosinate; protoporphyrinogen-IX oxidase inhibitors; lipid biosynthesis inhibitors such as acetyl CoA carboxylase (ACCase) inhibitors; or oxynil (i.e., bromoxynil or ioxynil) herbicides as a result of conventional methods of breeding or genetic engineering; furthermore, sulfonylurea-tolerant winter oilseed rape (winter canola) can have been made resistant to multiple classes of herbicides through multiple genetic modifications, such as resistance to both glyphosate and glufosinate or to both glyphosate and a herbicide from another class such as HPPD inhibitors, auxin herbicides, or ACCase inhibitors. These herbicide resistance technologies are, for example, described in Pest Management Science at volume, year, page 61, 2005, 246; 61, 2005, 258; 61, 2005, 277; 61, 2005, 269; 61, 2005, 286; 64, 2008, 326; 64, 2008, 332; Weed Science 57, 2009, 108; Australian Journal of Agricultural Research 58, 2007, 708; Science 316, 2007, 1185; and references quoted therein.

Furthermore, sulfonylurea-tolerant winter oilseed rape (winter canola) is described which is by the use of recombinant DNA techniques capable to synthesize one or more insecticidal proteins, especially those known from the bacterial genus *Bacillus,* particularly from *Bacillus thuringiensis,* such as δ-endotoxins, e.g. CryIA(b), CryIA(c), CryIF, CryIF(a2), CryIIA(b), CryIIIA, CryIIIB(b1) or Cry9c; vegetative insecticidal proteins (VIP), e.g. VIP1, VIP2, VIP3 or VIP3A; insecticidal proteins of bacteria colonizing nematodes, e.g. *Photorhabdus* spp. or *Xenorhabdus* spp.; toxins produced by animals, such as scorpion toxins, arachnid toxins, wasp toxins, or other insect-specific neurotoxins; toxins produced by fungi, such streptomycete toxins; plant lectins, such as pea or barley lectins; agglutinins; proteinase inhibitors, such as trypsin inhibitors, serine protease inhibitors, patatin, cystatin or papain inhibitors; ribosome-inactivating proteins (RIP), such as ricin, maize-RIP, abrin, luffin, saporin or bryodin; steroid metabolism enzymes, such as 3-hydroxy-steroid oxidase, ecdysteroid-IDP-glycosyl-transferase, cholesterol oxidases, ecdysone inhibitors or HMG-CoA-reductase; ion channel blockers, such as blockers of sodium or calcium channels; juvenile hormone esterase; diuretic hormone receptors (helicokinin receptors); stilben synthase, bibenzyl synthase, chitinases or glucanases. In the context of the present invention these insecticidal proteins or toxins are to be understood expressly also as pre-toxins, hybrid proteins, truncated or otherwise modified proteins. Hybrid proteins are characterized by a new combination of protein domains, (see, e.g. WO 02/015701). Further examples of such toxins or genetically modified plants capable of synthesizing such toxins are disclosed, e.g., in EP-A 374 753, WO 93/007278, WO 95/34656, EP-A 427 529, EP-A 451 878, WO 03/18810 und WO 03/52073. The methods for producing such genetically modified plants are generally known to the person skilled in the art and are described, e.g. in the publications mentioned above. These insecticidal proteins contained in the genetically modified plants impart to the plants producing these proteins tolerance to harmful pests from all taxonomic groups of arthropods, especially to beetles (*Coeloptera*), two-winged insects (*Diptera*), and moths (*Lepidoptera*) and to nematodes (*Nematoda*).

Furthermore, sulfonylurea-tolerant winter oilseed rape (winter canola) is described which is by the use of recombinant DNA techniques capable to synthesize one or more proteins to increase the resistance or tolerance of those plants to bacterial, viral or fungal pathogens. The methods for producing such genetically modified plants are generally known to the person skilled in the art.

Furthermore, sulfonylurea-tolerant winter oilseed rape (winter canola) is described which is by the use of recombinant DNA techniques capable to synthesize one or more proteins to increase the productivity (e.g. oil content), tolerance to drought, salinity or other growth-limiting environmental factors or tolerance to pests and fungal, bacterial or viral pathogens of those plants.

Furthermore, sulfonylurea-tolerant winter oilseed rape (winter canola) is described which contains by the use of recombinant DNA techniques a modified amount of substances of content or new substances of content, specifically to improve human or animal nutrition, e.g. oil crops that produce health-promoting long-chain omega-3 fatty acids or unsaturated omega-9 fatty acids (e.g. Nexera® rape, Dow Agro Sciences, Canada).

Agronomic products, for example, seed oil, seed meal, and the like are also described herein. Said agronomic products can be of feed quality or food quality. The agronomic products can be produced from plants, including seeds of said plants, treated by or obtained from the methods described throughout the detailed description herein.

### AHAS Enzymes

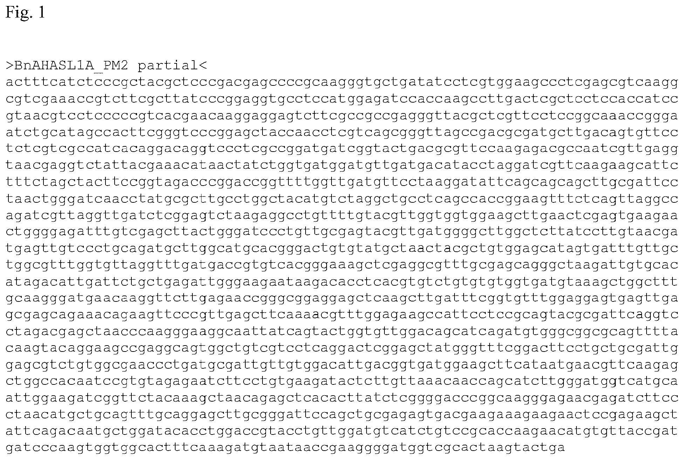

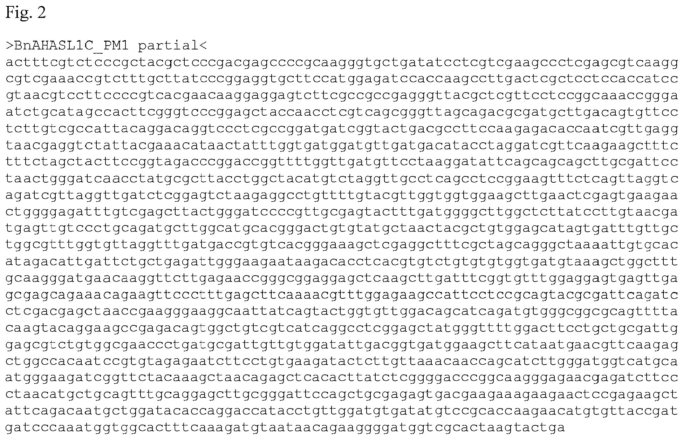

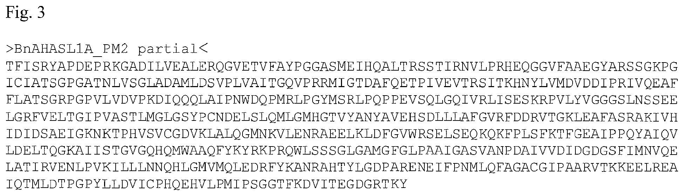

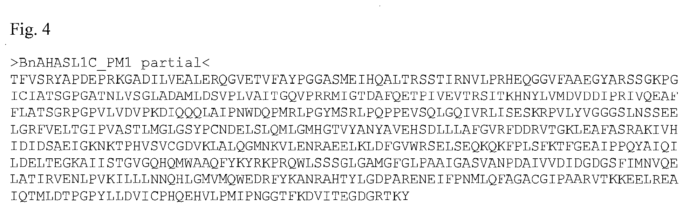

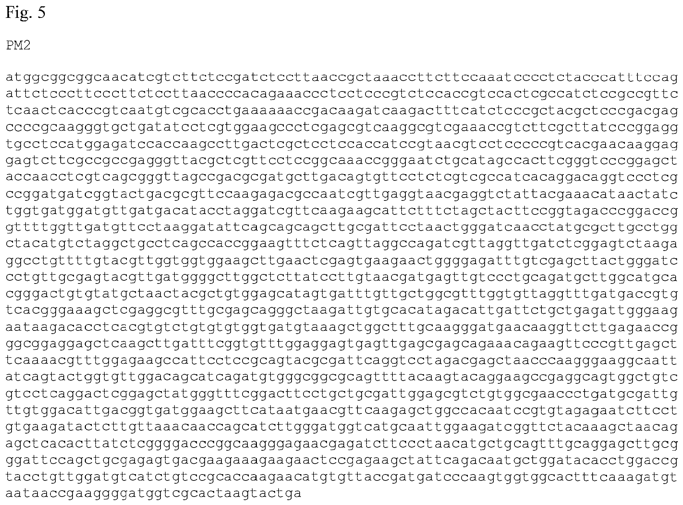

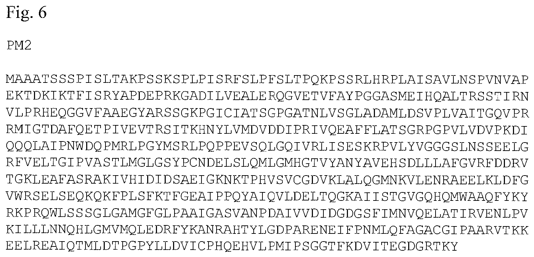

In various embodiments, winter-type *Brassica* plants containing both a W574(*At*)X and a S653(*At*)X in plastidic AHASL polypeptides thereof can be used. These can be present in different alleles, such as on different genomes, with each containing a single mutation in the respective AHASL gene, or these two can be present in a single AHASL, as a double-mutant allele. In various embodiments, these can be W574(*At*)L and S653(*At*)N: the former can be referred to as the "PM2" mutation and the latter as the "PM1" mutation. Fig. 1 (SEQ ID NO: 1) and Fig. 3 (SEQ ID NO:3) provide a partial nucleotide sequence and partial amino acid sequence, respectively, for the PM2 mutation in *B. napus.* Fig. 2 (SEQ ID NO:2) and Fig. 4 (SEQ ID NO:4) provide a partial nucleotide sequence and partial amino acid sequence, respectively, for the PM1 mutation in *B. napus.* Fig. 5 (SEQ ID NO:5) and Fig. 6 provide a second nucleotide sequence and second amino acid sequence, respectively, for the PM2 mutation, for example the PM2 sequence introgressed into *B. juncea* from *B. napus.*

The winter-type *Brassica* plants hereof can be inbred varieties, e.g., open-pollinated varieties, or hybrids, e.g., F1 hybrids.

Although transgenic or non-transgenic mutant AHAS traits can be employed herein, in winter-type *Brassica* crops, in various embodiments, the trait or traits can be non-transgenic, i.e., obtained by a process, excluding recombinant DNA techniques, and comprising mutagenesis, genoplasty, and/or isolation of spontaneous mutant plants. Many mutagenesis techniques are known in the art and these can involve application of a mutagenic chemical agent or radiation to seeds, plants parts, or cultured plant cells; alternatively, or in addition, the culturing of plant cells, or the conditions under which plant cells are cultured, can increase the rate of occurrence or accumulation of spontaneous mutations. Genoplasty techniques can include directed mutation-type strategies, such as methods comprising introduction, into the plant cell nucleus, of oligonucleotides that facilitate mismatch-repair-system-mediated nucleotide substitution.

Note that, in AHAS enzymes, there are two mutation sites known to be amenable to mutations that provide significant levels of tolerance to SUs. These both occur in AHASL at positions P197(*At*) and W574(*At*).

In various embodiments, the WOSR and other winter-type *Brassica* crops can contain one such mutation in the plastidic AHASL(s) thereof; in addition to one or more other mutations, in the same or different plastidic AHASL gene, that can be selected from those at sites where mutations have been found to be capable of providing tolerance toward one or more other AHAS inhibitor, examples of which sites include G121(*At*), A122(*At*), M124(*At*), R142(*At*), V196(*At*), R199(*At*), T203(*At*), A205(*At*), F206(*At*), K256(*At*), M351(*At*), H352(*At*), R373(*At*), D375(*At*), D376(*At*), R377(*At*), M570(*At*), V571(*At*), F578(*At*), S653(*At*), and G654(*At*).

Thus, in some embodiments, the WOSR and other winter-ty*pe Brassica* crops useful herein can contain one SU-HT mutation in the plastidic AHASL(s) thereof. In various embodiments, a plant useful herein can contain more than one herbicide tolerance mutation in the plastidic AHASL population therein. In various embodiments, these can be contained on different, single-mutant AHASL genes.

In sum, a WOSR or other winter-type *Brassica* crop useful herein can contain up to one expressible plastidic AHASL gene that encodes a mutation at W574(*At*), whether or not that AHASL gene also encodes other AHAS-inhibitor-tolerance mutation(s) [i.e., other than any additional mutation at a position selected from P197(*At*) or W574(*At*)], and whether or not that gene is represented by a single allele, as a heterozygote, or by two alleles, as a homozygote. Such WOSR or other *Brassica* crop does not contain more than one plastidic AHASL gene that encodes a mutation occurring at position W574(*At*) in the A genome.

When the WOSR or other winter-type *Brassica* crop contains an expressible plastidic AHASL gene encoding a mutation at W574(*At*), in a *Brassica* A-genome allele, then no such additional mutation is required to be present in an AHASL gene of the plant. Such embodiments would not include winter-type crops of *B. oleracea* (CC), *B. nigra* (BB), *B. carinata* (BBCC), and *Raphanobrassica* var. 'rabbage' (RRCC), which lack a *Brassica* A-genome.

As described herein, plants useful in various embodiments hereof contain one or more mutant AHASL gene(s) wherein at least one mutation therein confers herbicide tolerance to the AHAS enzyme of which the encoded AHASL is a part, and thereby confers herbicide tolerance to the plant in which it resides. Such a mutant AHASL gene is referred to as an "HT-AHASL gene". Also as described herein, plants useful in various embodiments hereof contain, as one such HT-AHASL gene, an SU-HT-AHASL gene, i.e., an HT-AHASL gene encoding a mutation that is W574X, such W574X mutations being referred to herein as sulfonylurea-tolerance HT mutations or "SU-HT" mutations. Plants hereof contain only one such SU-HT-AHASL gene, and this is a "mono-SU-HT-AHASL" gene. Said mono-SU-HT-AHASL gene is located in the *Brassica* A genome. As used herein, a "mono-SU-HT-AHASL" gene refers to an HT-AHASL gene that encodes only one SU-HT mutation, or only one SU-HT mutation per allele of said one gene. Thus, a "mono-SU-HT-AHASL" gene refers to an HT-AHASL gene that:
(1) encodes
   (a) only one of P197X or W574X and encodes it homozygously or hemizygously;
   (b) only one of P197X or W574X and encodes it in one allele and encodes a wild-type P197P or W574W residue, respectively, in the homologous allele;
   (c) two different P197X mutations heterozygously and is homozygous for wild-type W574W residues;
   (d) two different W574X mutations heterozygously and is homozygous for wild-type P197P residues; or
   (e) both (i) one P197X mutation and a wild-type W574W residue in one allele, and (ii) one W574X mutation and a wild-type P197P residue in the homologous allele; and
(2) optionally encodes Other HT mutation(s), as that term is defined herein.
In some embodiments, a mono-SU-HT-AHASL gene can encode no Other HT mutations.

The term "hemizygous" when used herein in regard to an AHASL mutation's being encoded "hemizygously" refers to the relationship between the corresponding loci of two homologous chromosomes in a genome, wherein one of the two loci is occupied by a (functioning) AHASL allele that contains the amino acid residue of the (substitution) mutation and the other locus either is occupied by a non-functioning AHASL allele or is unoccupied, e.g., the second allele being absent or having been deleted. Note that "mono-SU-HT-AHASL" genes listed under (1)(b)-(1)(e) above can be referred to as "heterozygous-mono-SU-HT-AHASL" genes, i.e., since they encode each SU-HT mutation heterozygously.

As described herein, *Brassica* plants in various embodiments hereof can contain at least one herbicide tolerant AHASL (HT-AHASL) gene, wherein only one of the HT-AHASL genes in the plant encodes a sulfonylurea herbicide tolerance (SU-HT) mutation that is W574X and is a mono-SU-HT-AHASL gene, which can optionally encode Other HT mutation(s), and wherein said mono-SU-HT-AHASL gene is located in the A genome of said *Brassica* plant.

In addition to being able to tolerate herbicides that inhibit AHAS activity, plants employed in methods of the invention may also be able to tolerate herbicides that work on other physiological processes. For example, the plants may be tolerant to acetyl-Coenzyme A carboxylase (ACCase) inhibitors, such as "dims" (e.g., cycloxydim, sethoxydim, clethodim, or tepraloxydim), "fops" (e.g., clodinafop, diclofop, fluazifop, haloxyfop, or quizalofop), and "dens" (such as pinoxaden); to inhibitors of 5-enolpyruvylshikimate-3-phosphate synthase (EPSPS) such as glyphosate; to inhibitors of protoporphyrinogen [IX] oxidase (PPO) such as saflufenacil; and to inhibitors of glutamine synthetase such as glufosinate. In addition to these classes of inhibitors, plants employed in methods of the invention may also be tolerant of herbicides having other modes of action, for example, auxin growth regulators (e.g., dicamba), chlorophyll/carotenoid pigment inhibitors, cell membrane destroyers, photosynthesis inhibitors, cell division inhibitors, root inhibitors, shoot inhibitors, and combinations thereof. Such tolerance traits may be expressed, e.g.: as mutant ACCase proteins, mutant EPSPS proteins, or mutant glutamine synthetase proteins; or as mutant native, inbred, or transgenic aryloxyalkanoate dioxygenase (AAD or DHT), haloarylnitrilase (BXN), 2,2-dichloropropionic acid dehalogenase (DEH), dicamba monooxygenase (DMO), glyphosate-N-acetyltransferase (GAT), glyphosate decarboxylase (GDC), glyphosate oxidoreductase (GOX), glutathione-S-transferase (GST), phosphinothricin acetyltransferase (PAT or bar), or cytochrome P450 (CYP450) proteins having an herbicide-degrading activity. Winter Brassica plants hereof can also be stacked with other traits including, but not limited to, pesticidal traits such as *Bt* Cry and other proteins having pesticidal activity toward coleopteran, lepidopteran, nematode, or other pests; nutrition or nutraceutical traits such as modified oil content or oil profile traits, high protein or high amino acid concentration traits, and other trait types known in the art.

Progeny of the herbicide-tolerant plants described herein of the invention as well as seeds and cells thereof are also described herein. Methods for producing seed by performing the methods described throughout the detailed description hereof and harvesting seed from the herbicide-tolerant plants are also described herein.
Seed can be harvested from *Brassica* plants treated by methods described throughout the detailed description.

### Nucleic acid molecules

Nucleic acid molecules that encode all or a portion of the AHASL proteins described above are also described herein. The nucleic acid molecules described herein can comprise a nucleic acid sequence encoding an amino acid sequence comprising a modified, or where applicable, unmodified, version of the sequences listed in the patent documents referenced in Table 2, wherein the resulting sequence encodes an AHASL protein that comprises one or more of the following: the amino acid at position 197 is other than proline while the amino acid at position 574 is tryptophan; or the amino acid at position 574 is other than tryptophan while the amino acid at position 197 is proline.

Nucleic acids that encode *Brassica* AHASLs having one or more Other HT-mutations are also described herein. Such AHASLs can also comprise amino acid sequences having one or more of the following: the amino acid at position 197 is other than proline while the amino acid at position 574 is tryptophan; or the amino acid at position 574 is other than tryptophan while the amino acid at position 197 is proline.

A nucleic acid molecule described herein can be DNA, derived from genomic DNA or cDNA, or RNA, . A nucleic acid molecule described herein can be naturally occurring or can be synthetic. A nucleic acid molecule described herein can be isolated, recombinant and/or mutagenized.

Nucleic acid molecules described herein can comprise non-coding sequences, which may or may not be transcribed. Such non-coding sequences include, but are not limited to, 5' and 3' UTRs, polyadenylation signals and regulatory sequences that control gene expression (e.g., promoters). Nucleic acid molecules described herein can also comprise sequences encoding transit peptides, protease cleavage sites, covalent modification sites and the like. Nucleic acid molecules described herein can encode a chloroplast transit peptide sequence in addition to a sequence encoding an AHAS enzyme.

Nucleic acid molecules described herein can encode an AHASL having at least 50%, 60%, 70%, 75%, 80%, 85%, 90%, 95% or more sequence identity to a P197X or W574X AHASL as described above, wherein the protein encoded by the sequence comprises one or more of the following: the amino acid at position 197 is other than proline while the amino acid at position 574 is tryptophan; or the amino acid at position 574 is other than tryptophan while the amino acid at position 197 is proline.

As used herein, "percent (%) sequence identity" is defined as the percentage of nucleotides or amino acids in the candidate derivative sequence identical with the nucleotides or amino acids in the subject sequence (or specified portion thereof), after aligning the sequences and introducing gaps, if necessary to achieve the maximum percent sequence identity, as generated by the program BLAST available at http://blast.ncbi.nlm.nih.gov/Blast.cgi with search parameters set to default values.

Also described herein are nucleic acid molecules that hybridize to nucleic acid molecules encoding a herein described AHAS enzyme as well as nucleic acid molecules that hybridize to the reverse complement of nucleic acid molecules encoding a herein described AHAS enzyme. The nucleic acid molecules can comprise nucleic acid molecules that hybridize to a nucleic acid molecule encoding a P197X or W574X AHASL as described above, wherein the protein encoded by the sequence comprises one or more of the following: the amino acid at position 197 is other than proline while the amino acid at position 574 is tryptophan; or the amino acid at position 574 is other than tryptophan while the amino acid at position 197 is proline as well as nucleic acid molecules complementary to all or a portion of the coding sequences, or the reverse complement of such nucleic acid molecules under stringent conditions. The stringency of hybridization can be controlled by temperature, ionic strength, pH, and the presence of denaturing agents such as formamide during hybridization and washing. Stringent conditions that can be used include those defined in Current Protocols in Molecular Biology, Vol. 1, Chap. 2.10, John Wiley & Sons, Publishers (1994) and Sambrook et al., Molecular Cloning, Cold Spring Harbor (1989).

The nucleic acid molecules described herein can encompass oligonucleotides that can be used as hybridization probes, sequencing primers, and/or PCR primers. Such oligonucleotides can be used, for example, to determine a codon sequence at a particular position in a nucleic acid molecule encoding an AHAS enzyme, for example, by allele specific PCR. Such oligonucleotides can be from about 15 to about 30, from about 20 to about 30, or from about 20-25 nucleotides in length.

### Herbicides

Herbicide compositions employed in various methods of the invention comprise one or more SU herbicides selected from the group consisting of amidosulfuron, flupyrsulfuron, foramsulfuron, imazosulfuron, iodosulfuron, mesosulfuron, nicosulfuron, thifensulfuron, and tribenuron, agronomically acceptable salts and esters thereof, and combinations thereof. In some embodiments, the herbicide composition can further comprise a significant amount of no other SU. Optionally, the herbicide compositions can further comprise A.I.(s) belonging to one or more additional classes of AHAS-inhibitor herbicides, e.g., imidazolinone herbicides, and/or one or more A.I. of other classes, e.g., agronomic fungicides, bactericides, algicides, nematicides, insecticides, and the like.

Each SU has its own recommended 1x dose rate. The 1x dose rates for SU active ingredients useful herein are shown below; these are also applicable to the salt or ester forms thereof.

**Table 3 Sulfonylurea herbicides and application rates**

| **Sulfonylurea(s)** | **1x (g/ha)** |
|---|---|
| Flupyrsulfuron | 10 |
| Imazosulfuron | 25 |
| Thifensulfuron | 30 |
| Tribenuron | 30 |
| Amidosulfuron | 30 |
| Foramsulfuron | 35 |
| Iodosulfuron | 10 |
| Mesosulfuron | 7.5 |
| Nicosulfuron | 30 |
| Mesosulfuron + lodosulfuron (5:1 w/w) | 14.4 |

Pre-emergent or pre-planting weed control methods useful in various embodiments hereof utilize >0.5x application rates of SU applied within about 30 days prior to emergence; in some embodiments, this can be ≥0.6x, ≥0.7x, ≥0.8x, ≥0.9x, or ≥1x of SU.

In addition, merely carry-over-tolerant WOSR plants have been found to lack tolerance to, or to exhibit insufficient tolerance, to post-emergent SU treatments. As a result, post-emergent weed control methods useful in various embodiments hereof utilize ≥0.25x application rates of SU; in some embodiments, this can be ≥0.3x, ≥0.4x, ≥0.5x, ≥0.6x, ≥0.7x, ≥0.8x, ≥0.9x, or ≥1x of SU.

Selection methods for herbicide tolerant winter *Brassica* plants also can be performed using these treatment method parameters, wherein no weeds are present in the immediate vicinity of the *Brassica* plant or its planting locus.

In either pre-emergent or post-emergent weed control methods hereof, the method can utilize 1x SU application rates with no significant injury to the plant; in some embodiments thereof, the application rate can exceed 1x SU; in some embodiments, the rate can be up to 4x SU, though more typically it will be about 2.5x or less, or about 2x or less. Where a combination of these SU active ingredients is employed, the herbicide application rate will preferably provide a summed rate that falls within the >0.5x to 4x or 0.25x to 4x SU range. For example, a 5:1 w/w combination of mesosulfuron and iodosulfuron having a 1x dose rate of 18 g/ha will, if applied at that rate, provide about 15 g/ha and 3 g/ha of these A.I.s, respectively: these are approximately 2x and 0.3x application rates, providing a summed rate of SU treatment of about 2.3x SU.

The herbicidal compositions hereof comprising a herbicide selected from the group of amidosulfuron, flupyrsulfuron, foramsulfuron, imazosulfuron, iodosulfuron, mesosulfuron, nicosulfuron, thifensulfuron, and tribenuron, agronomically acceptable salts and esters thereof, and combinations thereof, and optionally other agronomic A.I.(s), e.g., one or more imidazolinones selected from the group of imazamox, imazethapyr, imazapyr, imazapic, combinations thereof, and their agriculturally suitable salts and esters, can be used in any agronomically acceptable format. For example, these can be formulated as ready-to-spray aqueous solutions, powders, suspensions; as concentrated or highly concentrated aqueous, oily or other solutions, suspensions or dispersions; as emulsions, oil dispersions, pastes, dusts, granules, or other broadcastable formats. The herbicide compositions can be applied by any means known in the art, including, for example, spraying, atomizing, dusting, spreading, watering, seed treatment, or co-planting in admixture with the seed. The use forms depend on the intended purpose; in any case, they should ensure the finest possible distribution of the active ingredients.

Where the optional A.I. includes an AHAS-inhibitor, this can be selected from: (1) the imidazolinones, i.e., imazamox, imazethapyr, imazapyr, imazapic, imazaquin, and imazamethabenz, preferably from imazamox, imazethapyr, imazapyr, and imazapic, preferably imazamox; (2) the pyrimidinylbenzoates, i.e., including the pyrimidinyloxybenzoates (e.g., bispyribac, pyriminobac, and pyribenzoxim) and the pyrimidinylthiobenzoates (e.g., pyrithiobac and pyriftalid); and (3) the sulfonamides, i.e., including the sulfonylaminocarbonyltriazolinones (e.g., flucarbazone and propoxycarbazone) and the triazolopyrimidines (e.g., cloransulam, diclosulam, florasulam, flumetsulam, metosulam, and penoxsulam). The agronomically acceptable salts and esters of the foregoing are also included, as are combinations thereof.

Where the optional A.I. includes an herbicide from a different class to which the winter *Brassica* plant(s) hereof would normally be susceptible, the winter *Brassica* plant to be used is selected from among those that further comprise a trait of tolerance to such herbicide. Such further tolerance traits can be provided to the plant by any method known in the art, e.g., including techniques of traditional breeding to obtain a tolerance trait gene by hybridization or introgression, of mutagenesis, of genoplasty, and/or of transformation. Such plants can be described as having "stacked" traits.

Sulfonylurea herbicidal active ingredients useful in various embodiments hereof include those listed in Table 4.

**Table 4: Sulfonylurea Herbicide Active Ingredients**

| **SU A.I.** | **Example Salt or Ester** | **"Other SU"** |
|---|---|---|
| amidosulfuron | | - |
| azimsulfuron | | Other |
| bensulfuron | bensulfuron-methyl | Other |
| chlorimuron | chlorimuron-ethyl | Other |
| chlorsulfuron | | Other |
| cinosulfuron | | Other |
| cyclosulfamuron | | Other |
| ethametsulfuron | ethametsulfuron- methyl | Other |
| ethoxysulfuron | | Other |
| flazasulfuron | | Other |
| flucetosulfuron | | Other |
| flupyrsulfuron | flupyrsulfuron-methyl-sodium | - |
| foramsulfuron | | - |
| halosulfuron | halosulfuron-methyl | Other |
| imazosulfuron | | - |
| iodosulfuron | iodosulfuron-methyl-sodium | - |
| mesosulfuron | mesosulfuron- methyl | - |
| metazosulfuron | | Other |
| metsulfuron | metsulfuron-methyl | Other |
| nicosulfuron | | - |
| orthosulfamuron | | Other |
| oxasulfuron | | Other |
| primisulfuron | primisulfuron-methyl | Other |
| propyrisulfuron | | Other |
| prosulfuron | | Other |
| pyrazosulfuron | pyrazosulfuron-ethyl | Other |
| rimsulfuron | | Other |
| sulfometuron | sulfometuron- methyl | Other |
| sulfosulfuron | | Other |
| thifensulfuron | thifensulfuron-methyl | - |
| triasulfuron | | Other |
| tribenuron | tribenuron-methyl | - |
| trifloxysulfuron | trifloxysulfuron- sodium | Other |
| triflusulfuron | triflusulfuron-methyl | Other |
| tritosulfuron | | Other |

In some embodiments, an herbicide composition hereof that comprises a SU selected from the group consisting of amidosulfuron, flupyrsulfuron, foramsulfuron, imazosulfuron, iodosulfuron, mesosulfuron, nicosulfuron, thifensulfuron, and tribenuron, agronomically acceptable salts and esters thereof, and combinations thereof, optionally can further comprise a quantity, generally not more than 50% of the SU content of the composition, of one or more Other SU. As used herein "Other SU" refers to those SU A.I.s listed as "Other" in Table[4], along with their agronomically acceptable salts and esters, and combinations thereof.

In some embodiments, the Other SU content of the herbicide composition can be 50% or less by weight (wt.%) of the SU content of the composition, or about or less than 45%, 40%, 35%, 30%, 25%, 20%, 15%, 10%, 5%, 3%, 2%, or 1% by weight thereof; and, within that range, can be: 0 wt.% or more of the SU content of the composition, or about or more than 1%, 2%, 3%, 5%, 10%, 15%, 20%, 25%, 30%, 35%, 40%, or 45% by weight thereof.

In some embodiments, the Other SU content of the herbicide composition can be no more than a "substantial" amount, i.e., in this context meaning less than 50 wt.% of the SU content of the composition, e.g., from about 35 wt.% to less than 50 wt.%.

In some embodiments, the Other SU content of the herbicide composition can be no more than a "moderate" amount, i.e., in this context meaning about or less than 35 wt.% of the SU content of the composition, e.g., from about 20 wt.% to about 35 wt.%.

In some embodiments, the Other SU content of the herbicide composition can be no more than a "small" amount, i.e., in this context meaning about or less than 20 wt.% of the SU content of the composition, e.g., from about 10 wt.% to about 20 wt.%.

In some embodiments, the Other SU content of the herbicide composition can be no more than a "minor" amount, i.e., in this context meaning about or less than 10 wt.% of the SU content of the composition, e.g., from about 5 wt.% to about 10 wt.%.

In some embodiments, the Other SU content of the herbicide composition can be no more than a "minimal" amount, i.e., in this context meaning about or less than 5 wt.% of the SU content of the composition, e.g., from about 3 wt.% to about 5 wt.%.

In some embodiments, the Other SU content of the herbicide composition can be no more than a "significant" amount, i.e., in this context meaning about or less than 3 wt.% of the SU content of the composition, e.g., from about 1 wt.% to about 3 wt.%.

In some embodiments, the Other SU content of the herbicide composition can be no more than a "trace" amount, i.e., in this context meaning about or less than 1 wt.% of the SU content of the composition, e.g., from about 1 wt.% to greater than 0 wt.%.

In some embodiments, the Other SU content of the herbicide composition can be 0 wt.% or can be about 0 wt.%, e.g., from about 0.5 wt.% to 0 wt.%.

In the above list of ranges of Other SU content of the herbicide composition, in those jurisdictions in which the term "about" is impermissible, this list of ranges is to be read without said term. In any remaining portion of the Description in which the term "about" is used, the Description is to be read without said term in those jurisdictions in which the term "about" is impermissible.

Optional A.I.s of other herbicide classes include ACCase inhibitors, PPO inhibitors, EPSPS inhibitors, glutamine synthetase inhibitors, p-hydroxyphenylpyruvate dioxygenase (4-HPD) inhibitors. Optional A.I.s of other types include, but are not limited to fungicides such as strobilurins, e.g., pyraclostrobin; insecticides such as nematicides, lepidoptericides, coleoptericides; molluskicides, and others known in the art.

The herbicidal compositions comprising a herbicide selected from the group of amidosulfuron, flupyrsulfuron, foramsulfuron, imazosulfuron, iodosulfuron, mesosulfuron, nicosulfuron, thifensulfuron, and tribenuron, agronomically acceptable salts and esters thereof, and combinations thereof, and optionally other agronomic A.I.(s), e.g., one or more imidazolinones selected from the group of imazamox, imazethapyr, imazapyr, imazapic, combinations thereof, and their agriculturally suitable salts and esters can also comprise auxiliaries which are customary for the formulation of crop protection agents.

Examples of auxiliaries customary for the formulation of crop protection agents include inert auxiliaries, solid carriers, surfactants (such as dispersants, protective colloids, emulsifiers, wetting agents and tackifiers), organic and inorganic thickeners, penetrants (such as penentration-enhancing organosilicone surfactants or acidic sulfate chelates, e.g., CT-301™ available from Cheltec, Inc.), safeners, bactericides, antifreeze agents, antifoams, colorants, and adhesives. Formulations of the herbicide compositions useful herein can be prepared according to any method known useful therefor in the art

Examples of thickeners (i.e., compounds which impart to the formulation modified flow properties, i.e., high viscosity in the state of rest and low viscosity in motion) are polysaccharides, such as xanthan gum (Kelzan® from Kelco), Rhodopol® 23 (Rhone Poulenc) or Veegum® (from R.T. Vanderbilt), and also organic and inorganic sheet minerals, such as Attaclay® (from Engelhardt).

Examples of antifoams are silicone emulsions (such as, for example, Silikon® SRE, Wacker or Rhodorsil® from Rhodia), long-chain alcohols, fatty acids, salts of fatty acids, organofluorine compounds and mixtures thereof.

Bactericides can be added for stabilizing the aqueous herbicidal formulations. Examples of bactericides are bactericides based on diclorophen and benzyl alcohol hemiformal (Proxel® from ICI or Acticide® RS from Thor Chemie and Kathon® MK from Rohm & Haas), and also isothiazolinone derivates, such as alkylisothiazolinones and benzisothiazolinones (Acticide MBS from Thor Chemie).

Examples of antifreeze agents are ethylene glycol, propylene glycol, urea or glycerol.

Examples of colorants include members of colorant classes such as the sparingly water-soluble pigments and the water-soluble dyes. Some specific examples of these include the dyes known under the names Rhodamin B, C.I. Pigment Red 112 and C.I. Solvent Red 1, and also pigment blue 15:4, pigment blue 15:3, pigment blue 15:2, pigment blue 15:1, pigment blue 80, pigment yellow 1, pigment yellow 13, pigment red 112, pigment red 48:2, pigment red 48:1, pigment red 57:1, pigment red 53:1, pigment orange 43, pigment orange 34, pigment orange 5, pigment green 36, pigment green 7, pigment white 6, pigment brown 25, basic violet 10, basic violet 49, acid red 51, acid red 52, acid red 14, acid blue 9, acid yellow 23, basic red 10, basic red 108.

Examples of adhesives are polyvinylpyrrolidone, polyvinyl acetate, polyvinyl alcohol and tylose.

Suitable inert auxiliaries are, for example, the following:

mineral oil fractions of medium to high boiling point, such as kerosene and diesel oil, furthermore coal tar oils and oils of vegetable or animal origin, aliphatic, cyclic and aromatic hydrocarbons, for example paraffin, tetrahydronaphthalene, alkylated naphthalenes and their derivatives, alkylated benzenes and their derivatives, alcohols such as methanol, ethanol, propanol, butanol and cyclohexanol, ketones such as cyclohexanone or strongly polar solvents, for example amines such as N-methylpyrrolidone, and water.

Suitable carriers include liquid and solid carriers.

Liquid carriers include e.g. non-aqueous solvents such as cyclic and aromatic hydrocarbons, e.g. paraffins, tetrahydronaphthalene, alkylated naphthalenes and their derivatives, alkylated benzenes and their derivatives, alcohols such as methanol, ethanol, propanol, butanol and cyc-lohexanol, ketones such as cyclohexanone, strongly polar solvents, e.g. amines such as N-methylpyrrolidone, and water as well as mixtures thereof.

Solid carriers include e.g. mineral earths such as silicas, silica gels, silicates, talc, kaolin, limestone, lime, chalk, bole, loess, clay, dolomite, diatomaceous earth, calcium sulfate, magnesium sulfate and magnesium oxide, ground synthetic materials, fertilizers such as ammonium sulfate, ammonium phosphate, ammonium nitrate and ureas, and products of vegetable origin, such as cereal meal, tree bark meal, wood meal and nutshell meal, cellulose powders, or other solid carriers.

Suitable surfactants (adjuvants, wetting agents, tackifiers, dispersants and also emulsifiers) are the alkali metal salts, alkaline earth metal salts and ammonium salts of aromatic sulfonic acids, for example lignosulfonic acids (e.g. Borrespers-types, Borregaard), phenolsulfonic acids, naphthalenesulfonic acids (Morwet types, Akzo Nobel) and dibutylnaphthalenesulfonic acid (Nekal types, BASF AG), and of fatty acids, alkyl- and alkylarylsulfonates, alkyl sulfates, lauryl ether sulfates and fatty alcohol sulfates, and salts of sulfated hexa-, hepta- and octadecanols, and also of fatty alcohol glycol ethers, condensates of sulfonated naphthalene and its derivatives with formaldehyde, condensates of naphthalene or of the naphthalenesulfonic acids with phenol and formaldehyde, polyoxyethylene octylphenol ether, ethoxylated isooctyl-, octyl- or nonylphenol, alkylphenyl or tributylphenyl polyglycol ether, alkylaryl polyether alcohols, isotridecyl alcohol, fatty alcohol/ethylene oxide condensates, ethoxylated castor oil, polyoxyethylene alkyl ethers or polyoxypropylene alkyl ethers, lauryl alcohol polyglycol ether acetate, sorbitol esters, lignosulfite waste liquors and proteins, denatured proteins, polysaccharides (e.g. methylcellulose), hydrophobically modified starches, polyvinyl alcohol (Mowiol types, Clariant), polycarboxylates (BASF AG, Sokalan types), polyalkoxylates, polyvinylamine (BASF AG, Lupamine types), polyethyleneimine (BASF AG, Lupasol types), polyvinylpyrrolidone and copolymers thereof.

Powders, materials for broadcasting and dusts can be prepared by mixing or concomitant grinding the active ingredients together with a solid carrier.

Granules, for example coated granules, impregnated granules and homogeneous granules, can be prepared by binding the active ingredients to solid carriers.

Aqueous use forms can be prepared from emulsion concentrates, suspensions, pastes, wettable powders or water-dispersible granules by adding water.

To prepare emulsions, pastes or oil dispersions, the herbicidal compositions comprising a herbicide selected from the group of amidosulfuron, flupyrsulfuron, foramsulfuron, imazosulfuron, iodosulfuron, mesosulfuron, nicosulfuron, thifensulfuron, and tribenuron, agronomically acceptable salts and esters thereof, and combinations thereof, and optionally other agronomic A.I.(s), e.g., one or more imidazolinones selected from the group of imazamox, imazethapyr, imazapyr, imazapic, combinations thereof, and their agriculturally suitable salts and esters, either as such or dissolved in an oil or solvent, can be homogenized in water by means of a wetting agent, tackifier, dispersant or emulsifier. Alternatively, it is also possible to prepare concentrates comprising active compound, wetting agent, tackifier, dispersant or emulsifier and, if desired, solvent or oil, which are suitable for dilution with water.

The concentrations of the herbicides selected from the group of amidosulfuron, flupyrsulfuron, foramsulfuron, imazosulfuron, iodosulfuron, mesosulfuron, nicosulfuron, thifensulfuron, and tribenuron, agronomically acceptable salts and esters thereof, and combinations thereof, and optionally other agronomic A.I.(s), e.g., one or more imidazolinones selected from the group of imazamox, imazethapyr, imazapyr, imazapic, combinations thereof, and their agriculturally suitable salts and esters in the ready-to-use preparations (formulations) can be varied within wide ranges. In general, the formulations comprise approximately from 0.001 to 98% by weight, preferably 0.01 to 95% by weight of at least one active ingredient. The active ingredients are employed in a purity of from 90% to 100%, preferably 95% to 100% (according to NMR spectrum).

In the formulation of the herbicides selected from the group of amidosulfuron, flupyrsulfuron, foramsulfuron, imazosulfuron, iodosulfuron, mesosulfuron, nicosulfuron, thifensulfuron, and tribenuron, agronomically acceptable salts and esters thereof, and combinations thereof, and optionally other agronomic A.I.(s), e.g., one or more imidazolinones selected from the group of imazamox, imazethapyr, imazapyr, imazapic, combinations thereof, and their agriculturally suitable salts and esters the active ingredients, e.g. the herbicides selected from the group of amidosulfuron, flupyrsulfuron, foramsulfuron, imazosulfuron, iodosulfuron, mesosulfuron, nicosulfuron, thifensulfuron, and tribenuron, agronomically acceptable salts and esters thereof, and combinations thereof, and optionally other agronomic A.I.(s), e.g., one or more imidazolinones selected from the group of imazamox, imazethapyr, imazapyr, imazapic, combinations thereof and their agriculturally suitable salts and esters, are present in suspended, emulsified or dissolved form. The formulation can be in the form of aqueous solutions, powders, suspensions, also highly-concentrated aqueous, oily or other suspensions or dispersions, aqueous emulsions, aqueous microemulsions, aqueous suspo-emulsions, oil dispersions, pastes, dusts, materials for spreading or granules.

The herbicides selected from the group of amidosulfuron, flupyrsulfuron, foramsulfuron, imazosulfuron, iodosulfuron, mesosulfuron, nicosulfuron, thifensulfuron, and tribenuron, agronomically acceptable salts and esters thereof, and combinations thereof and optionally other agronomic A.I.(s), e.g., one or more imidazolinones selected from the group of imazamox, imazethapyr, imazapyr, imazapic, combinations thereof, and their agriculturally suitable salts and esters can, for example, be formulated as follows:

### 1. Products for dilution with water

### A Water-soluble concentrates

10 parts by weight of active compound are dissolved in 90 parts by weight of water or a water-soluble solvent. As an alternative, wetting agent(s) or other adjuvants are added. The active compound dissolves upon dilution with water. This gives a formulation with an active compound content of 10% by weight.

### B Dispersible concentrates

20 parts by weight of active compound are dissolved in 70 parts by weight of cyclohexanone with addition of 10 parts by weight of a dispersant, for example polyvinylpyrrolidone. Dilution with water gives a dispersion. The active compound content is 20% by weight.

### C Emulsifiable concentrates

15 parts by weight of active compound are dissolved in 75 parts by weight of an organic solvent (e.g. alkylaromatics) with addition of calcium dodecylbenzenesulfonate and castor oil ethoxylate (in each case 5 parts by weight). Dilution with water gives an emulsion. The formulation has an active compound content of 15% by weight.

### D Emulsions

25 parts by weight of active compound are dissolved in 35 parts by weight of an organic solvent (e.g. alkylaromatics) with addition of calcium dodecylbenzenesulfonate and castor oil ethoxylate (in each case 5 parts by weight). This mixture is introduced into 30 parts by weight of water by means of an emulsifier (Ultraturrax) and made into a homogeneous emulsion. Dilution with water gives an emulsion. The formulation has an active compound content of 25% by weight.

### E Suspensions

In an agitated ball mill, 20 parts by weight of active compound are comminuted with addition of 10 parts by weight of dispersants and wetting agent(s) and 70 parts by weight of water or an organic solvent to give a fine active compound suspension. Dilution with water gives a stable suspension of the active compound. The active compound content in the formulation is 20% by weight.

### F Water-dispersible granules and water-soluble granules

50 parts by weight of active compound are ground finely with addition of 50 parts by weight of dispersants and wetting agent(s) and made into water-dispersible or water-soluble granules by means of technical appliances (for example extrusion, spray tower, fluidized bed). Dilution with water gives a stable dispersion or solution of the active compound. The formulation has an active compound content of 50% by weight.

### G Water-dispersible powders and water-soluble powders

75 parts by weight of active compound are ground in a rotor-stator mill with addition of 25 parts by weight of dispersants, wetting agent(s) and silica gel. Dilution with water gives a stable dispersion or solution of the active compound. The active compound content of the formulation is 75% by weight.

### H Gel formulations

In a ball mill, 20 parts by weight of active compound, 10 parts by weight of dispersant, 1 part by weight of gelling agent and 70 parts by weight of water or of an organic solvent are mixed to give a fine suspension. Dilution with water gives a stable suspension with active compound content of 20% by weight.

### 2. Products to be applied undiluted

### I Dusts

5 parts by weight of active compound are ground finely and mixed intimately with 95 parts by weight of finely divided kaolin. This gives a dusting powder with an active compound content of 5% by weight.

### J Granules (GR, FG, GG, MG)

0.5 parts by weight of active compound are ground finely and associated with 99.5 parts by weight of carriers. Current methods here are extrusion, spray-drying or the fluidized bed. This gives granules to be applied undiluted with an active compound content of 0.5% by weight.

### K ULV solutions (UL)

10 parts by weight of active compound are dissolved in 90 parts by weight of an organic solvent, for example xylene. This gives a product to be applied undiluted with an active compound content of 10% by weight.

Aqueous use forms can be prepared from emulsion concentrates, suspensions, pastes, wettable powders or water-dispersible granules by adding water.

The herbicides selected from the group of amidosulfuron, flupyrsulfuron, foramsulfuron, imazosulfuron, iodosulfuron, mesosulfuron, nicosulfuron, thifensulfuron, and tribenuron, agronomically acceptable salts and esters thereof, and combinations thereof, and optionally other agronomic A.I.(s), e.g., one or more imidazolinones selected from the group of imazamox, imazethapyr, imazapyr, imazapic, combinations thereof, and their agriculturally suitable salts and esters or the herbicidal compositions comprising them can be applied pre-, post-emergence or pre-plant, or together with the seed of the imidazolinone-resistant winter oilseed rape plant. It is also possible to apply the herbicidal composition or active compounds by applying seed, pretreated with the herbicidal compositions or active compounds, of a crop plant.

In a further embodiment, the herbicides selected from the group of amidosulfuron, flupyrsulfuron, foramsulfuron, imazosulfuron, iodosulfuron, mesosulfuron, nicosulfuron, thifensulfuron, and tribenuron, agronomically acceptable salts and esters thereof, and combinations thereof, and optionally other agronomic A.I.(s), e.g., one or more imidazolinones selected from the group of imazamox, imazethapyr, imazapyr, imazapic, combinations thereof, and their agriculturally suitable salts and esters or the herbicidal compositions can be applied by treating seed. The treatment of seeds comprises essentially all procedures familiar to the person skilled in the art (seed dressing, seed coating, seed dusting, seed soaking, seed film coating, seed multilayer coating, seed encrusting, seed dripping and seed pelleting) based on the herbicides selected from the group of amidosulfuron, flupyrsulfuron, foramsulfuron, imazosulfuron, iodosulfuron, mesosulfuron, nicosulfuron, thifensulfuron, and tribenuron, agronomically acceptable salts and esters thereof, and combinations thereof, and optionally other agronomic A.I.(s), e.g., one or more imidazolinones selected from the group of imazamox, imazethapyr, imazapyr, imazapic, combinations thereof, and their agriculturally suitable salts and esters or the compositions prepared therefrom. Here, the herbicidal compositions can be applied diluted or undiluted.

The term "seed" comprises seed of all types, such as, for example, corns, seeds, fruits, tubers, seedlings and similar forms. Here, preferably, the term seed describes corns and seeds.

The seed used can be seed of the useful plants mentioned above, but also the seed of transgenic plants or plants obtained by customary breeding methods.

To widen the spectrum of action, the SU herbicide A.I.(s) selected from the group of amidosulfuron, flupyrsulfuron, foramsulfuron, imazosulfuron, iodosulfuron, mesosulfuron, nicosulfuron, thifensulfuron, and tribenuron, agronomically acceptable salts and esters thereof and combinations thereof, and optionally other agronomic A.I.(s), e.g., one or more imidazolinones selected from the group of imazamox, imazethapyr, imazapyr, imazapic, combinations thereof, and their agriculturally suitable salts and esters, can be mixed with a large number of representatives of other herbicidal or growth-regulating active ingredient groups and then applied concomitantly. Suitable components for mixtures are, for example, 1,2,4-thiadiazoles, 1,3,4-thiadiazoles, amides, aminophosphoric acid and its derivatives, aminotriazoles, anilides, (het)aryloxyalkanoic acids and their derivatives, benzoic acid and its derivatives, benzothiadiazinones, 2-aroyl-1,3-cyclohexanediones, 2-hetaroyl-1,3-cyclohexane-diones, hetaryl aryl ketones, benzylisoxazolidinones, meta-CF3-phenyl derivatives, carbamates, quinolinecarboxylic acid and its derivatives, chloroacetanilides, cyclohexenone oxime ether derivatives, diazines, dichloropropionic acid and its derivatives, dihydro-benzofurans, dihydrofuran-3-ones, dinitroanilines, dinitrophenols, diphenyl ethers, dipyridyls, halocarboxylic acids and their derivatives, ureas, 3 phenyluracils, imidazoles, imidazolinones, N-phenyl-3,4,5,6-tetrahydrophthalimides, oxadiazoles, oxiranes, phenols, aryloxy- and hetaryloxyphenoxypropionic esters, phenylacetic acid and its derivatives, 2-phenylpropionic acid and its derivatives, pyrazoles, phenylpyrazoles, pyridazines, pyridinecarboxylic acid and its derivatives, pyrimidyl ethers, sulfonamides, sulfonylureas, triazines, triazinones, triazolinones, triazolecarboxamides, uracils, phenyl pyrazolines and isoxazolines and derivatives thereof.

It may furthermore be beneficial to apply the herbicides selected from the group of amidosulfuron, flupyrsulfuron, foramsulfuron, imazosulfuron, iodosulfuron, mesosulfuron, nicosulfuron, thifensulfuron, and tribenuron, agronomically acceptable salts and esters thereof, and combinations thereof, and optionally other agronomic A.I.(s), e.g., one or more imidazolinones selected from the group of imazamox, imazethapyr, imazapyr, imazapic, combinations thereof, and their agriculturally suitable salts and esters alone or in combination with other herbicides, or else in the form of a mixture with other crop protection agents, for example together with agents for controlling pests or phytopathogenic fungi or bacteria. Also of interest is the miscibility with mineral salt solutions, which are employed for treating nutritional and trace element deficiencies. Other additives such as non-phytotoxic oils and oil concentrates can also be added.

Moreover, it may be useful to apply the herbicides selected from the group of amidosulfuron, flupyrsulfuron, foramsulfuron, imazosulfuron, iodosulfuron, mesosulfuron, nicosulfuron, thifensulfuron, and tribenuron, agronomically acceptable salts and esters thereof, and combinations thereof, and optionally other agronomic A.I.(s), e.g., one or more imidazolinones selected from the group of imazamox, imazethapyr, imazapyr, imazapic, combinations thereof, and their agriculturally suitable salts and esters, in combination with safeners. Safeners are chemical compounds which prevent or reduce herbicide-induced injury to useful plants without having a major impact on the herbicidal action of the herbicides amidosulfuron, flupyrsulfuron, foramsulfuron, imazosulfuron, iodosulfuron, mesosulfuron, nicosulfuron, thifensulfuron, and tribenuron, agronomically acceptable salts and esters thereof, and combinations thereof, and optionally other agronomic A.I.(s), e.g., one or more imidazolinones selected from the group of imazamox, imazethapyr, imazapyr, imazapic, combinations thereof and their agriculturally suitable salts and esters, towards unwanted plants. They can be applied either before sowings (e.g. on seed treatments, shoots or seedlings) or in the pre-emergence application or post-emergence application of the useful plant. The safeners and the herbicides amidosulfuron, flupyrsulfuron, foramsulfuron, imazosulfuron, iodosulfuron, mesosulfuron, nicosulfuron, thifensulfuron, and tribenuron, agronomically acceptable salts and esters thereof, and combinations thereof, and optionally other agronomic A.I.(s), e.g., one or more imidazolinones selected from the group of imazamox, imazethapyr, imazapyr, imazapic, combinations thereof, and their agriculturally suitable salts and esters, can be applied simultaneously or in succession.

Suitable safeners are e.g. (quinolin-8-oxy)acetic acids, 1-phenyl-5-haloalkyl-1H-1,2,4-triazol-3-carboxylic acids, 1-phenyl-4,5-dihydro-5-alkyl-1H-pyrazol-3,5-dicarboxylic acids, 4,5-dihydro-5,5-diaryl-3-isoxazol carboxylic acids, dichloroacetamides, alpha-oximinophenylacetonitriles, acetophenonoximes, 4,6-dihalo-2-phenylpyrimidines, N-[[4-(aminocarbonyl)phenyl]sulfonyl]-2-benzoic amides, 1,8-naphthalic anhydride, 2-halo-4-(haloalkyl)-5-thiazol carboxylic acids, phosphorthiolates and N-alkyl-O-phenyl-carbamates and their agriculturally acceptable salts and their agriculturally acceptable derivatives such amides, esters, and thioesters, provided they have an acid group.

### Methods of controlling weeds

Herbicide-tolerant plants described herein can be used in conjunction with an herbicide to which they are tolerant. Herbicides can be applied to the plants using any techniques known to those skilled in the art. Herbicides can be applied at any point in the plant cultivation process. For example, herbicides can be applied pre-planting, at planting, pre-emergence, post-emergence or combinations thereof.

Herbicide compositions hereof can be applied, e.g., as foliar treatments, soil treatments, seed treatments, or soil drenches. Application can be made, e.g., by spraying, dusting, broadcasting, or any other mode known useful in the art.

Herbicides can be used to control the growth of weeds that may be found growing in the vicinity of the herbicide-tolerant plants described herein. For example, an herbicide can be applied to a plot in which the herbicide-tolerant plants are growing in vicinity to weeds. An herbicide to which the herbicide-tolerant plant is tolerant can then be applied to the plot at a concentration sufficient to kill or inhibit the growth of the weed. Concentrations of herbicide sufficient to kill or inhibit the growth of weeds are known in the art and are disclosed above.

The methods of controlling weeds can also include a step of selecting a winter-type *Brassica* plant capable of tolerating the SU herbicide composition. As used herein, a step of selecting a winter-type *Brassica* plant capable of tolerating the SU herbicide composition can be performed by a person's choosing the plant to be grown, or by a first person's choosing to have a second person choose the plant to be grown. For example, a *Brassica* producer may be operating as a seed multiplier to produce seed, or operating as a grain grower to produce grain or other produce for market. In either situation, the *Brassica* producer can choose for himself what *Brassica* variety to grow, or can permit another to choose what *Brassica* variety he will grow, or can have pre-chosen by prior contractual arrangement to grow a *Brassica* variety to be chosen by a third party, e.g., pursuant to a service agreement, a forward contract, or other arrangement. All such modes by which a *Brassica* producer chooses what *Brassica* variety to grow can constitute a step of selecting a winter-type *Brassica* plant hereof.

### Methods of Providing Yield Protection

Methods of planting, growing and treating with SU herbicide compositions winter-type *Brassica* plants according to various embodiments of the present invention can provide yield protection to a winter-type *Brassica* crop grown in the presence of a sulfonylurea (SU) herbicide composition. The methods can comprise:
planting a seed of a winter-type *Brassica* plant in the presence of the SU herbicide composition described herein; and
growing the seed under conditions which will produce the winter-type *Brassica* plant;
wherein said *Brassica* plant comprises at least one herbicide tolerant AHASL (HT-AHASL) gene, wherein only one of the HT-AHASL genes in the plant encodes a sulfonylurea herbicide tolerance (SU-HT) mutation that is W574X and is a mono-SU-HT-AHASL gene, which can optionally encode Other HT mutation(s), and wherein said mono-SU-HT-AHASL gene is located in the A genome of said *Brassica*;
wherein the yield can be equal to or greater than that provided by a wild-type version of the same type of winter-type *Brassica* plant.

These methods can also comprise:
planting a seed of a winter-type *Brassica* plant;
growing the seed under conditions which will produce the winter-type *Brassica* plant; and
performing an herbicide treatment of the plant by applying a herein described herbicide composition, comprising sulfonylurea(s) (SU), to the plant and its immediate vicinity; wherein said *Brassica* plant comprises at least one herbicide tolerant AHASL (HT-AHASL) gene, wherein only one of the HT-AHASL genes in the plant encodes a sulfonylurea herbicide tolerance (SU-HT) mutation that is W574X and is a mono-SU-HT-AHASL gene, which can optionally encode Other HT mutation(s), and wherein said mono-SU-HT-AHASL gene is located in the A genome of said *Brassica*;
wherein the yield can be equal to or greater than that provided by a wild-type version of the same type of winter-type *Brassica* plant. The herbicide treatment may be a pre-flowering treatment of the plant.

The methods of providing yield protection can also include a step of choosing a winter-type *Brassica* plant capable of tolerating the SU herbicide composition.

The methods of providing yield protection can further comprise harvesting seeds produced by the winter-type *Brassica* plants. These methods can also control weeds in the vicinity of the winter-type *Brassica* plants.

As used herein, "yield protection" includes, but is not limited to, a reduced risk of crop loss, reduction in yield or both. Negative effects of SU herbicide compositions on winter-type *Brassica* plants can include, but are not limited to, death; transient plant injury; delayed growth; altered maturation; significant visual injury symptoms; decreases in field plant density (i.e., fewer plants in the population); and increases in the proportion of plants exhibiting delayed-maturation, smaller stature (less biomass) and/or injury from disease or insect attack beginning during periods of temporary metabolic stress/wilt phase (i.e., transitory SU herbicide injury). Non-SU-tolerant winter-type *Brassica* plants typically suffer from negative effects in the presence of SU herbicide compositions; and winter-type *Brassica* plants that are tolerant solely to residual amounts of SU herbicides can also suffer from negative effects. However, the winter-type *Brassica* plants employed in methods of the invention are more robust to exposure to such SU herbicide compositions. Thus, a winter-type *Brassica* crop grown from such SU-tolerant winter-type *Brassica* plants can provide a greater yield than such SU herbicide susceptible or residual tolerant plants grown in the presence of SU herbicide compositions.

A number of desires can motivate the step of choosing a winter-type *Brassica* plant capable of tolerating SU herbicide compositions. For example, and without limitation, a *Brassica* producer may desire: (1) to control *Brassica* crop weeds using an SU application, wherein an SU herbicide application could not otherwise be applied to such *Brassica* crop without substantial crop injury or loss; or (2) to avoid or decrease the risk of permanent or transient crop injury from SU residues in soil, or to avoid or decrease such risk better than can use of a *Brassica* that is only SU-residue-tolerant; or (3) to avoid or decrease the risk of permanent or transient crop injury from SU residues present in tanks re-used for preparing or supplying other agronomic products to the crop, or to avoid or decrease such risk better than can use of a *Brassica* that is only SU-residue-tolerant. Choosing winter-type *Brassica* plants described herein can achieve these goals.

A Brassica producer may desire to control *Brassica* crop weeds using an SU application. Among the *Brassica* crop weeds that can be treated are, e.g., *Brassicaceae* family weeds such as Wild turnip (Brassica tournefortii), Shepherd's purse (Capsella bursa-pastoris), Hare's ear mustard (Conringia orientalis), Wormseed mustard (Erysimum cheiranthoides; Treacle mustard), Buchan weed (Hirschfeldia incana), Common peppergrass (Lepidium virginicum; Virginia pepperweed), Musk weed (Myagrum perfoliatum), Ball mustard (Neslia paniculata), Wild radish (Raphanus raphanistrum), Turnip weed (Rapistrum rugosum), Wild mustard (Sinapis arvensis; Charlock), Indian hedge mustard (Sisymbrium orientale), Flixweed (Sisymbrium sophia; Tansy mustard; Fluxweed), and Stinkweed (Thlaspi arvense; Field pennycress). Such *Brassicacea* family weeds can be problematic to control in traditional *Brassica* crops. Choosing winter-type *Brassica* plants described herein can achieve these goals.

As used herein, a step of choosing a winter-type *Brassica* plant capable of tolerating the SU herbicide composition can be performed by a person's choosing the plant to be grown, or by a first person's choosing to have a second person choose the plant to be grown. For example, a *Brassica* producer may be operating as a seed multiplier to produce seed, or operating as a grain grower to produce grain or other produce for market. In either situation, the *Brassica* producer can choose for himself what *Brassica* variety to grow, or can permit another to choose what *Brassica* variety he will grow, or can have pre-chosen by prior contractual arrangement to grow a *Brassica* variety to be chosen by a third party, e.g., pursuant to a service agreement, a forward contract, or other arrangement. All such modes by which a *Brassica* producer chooses what *Brassica* variety to grow can constitute a step of choosing a winter-type *Brassica* plant hereof.

A crop of the winter-type *Brassica* plants described herein which is grown in soil containing SU herbicides, and optionally containing sulfonamide and/or imidazolinone AHAS inhibiting herbicides, can achieve a higher yield than a crop of winter-type *Brassica* plants of the corresponding wild-type isoline grown in the same herbicide containing conditions. Additionally, a crop grown of the winter-type *Brassica* plants described herein which is exposed to SU herbicides from sprayers contaminated with SU herbicides from leftovers in herbicide mixing tanks provide a yield protection benefit compared to winter-type *Brassica* plants of the corresponding wild-type isoline.

Crops of winter-type *Brassica* plants described herein can produce substantially equivalent yields when exposed to SU herbicides and when not exposed to SU herbicides, when grown under otherwise similar conditions. Additionally, crops of the winter-type *Brassica* plants can achieve equal yields when exposed to SU herbicides from contaminated sprayers and when not exposed to SU herbicides from contaminated sprayers, when grown under otherwise similar conditions.

Winter-type *Brassica* Crop Containing AHASL Gene Encoding Mutation at P 197(*At*) or W574(*At*) and Located in Any *Brassica* Genome.

Winter-type *Brassica* plants having an expressible plastidic AHASL gene that encodes a mutation at P197(*At*) or W574(*At*) located in any genome, for example a *Brassica* A-, B- or C-genome are also described herein. This is an exception to the above-described *Brassica* plants where the herbicide-tolerant AHASL gene can be located only in the *Brassica* A genome. Plants in which the WOSR or other winter-type *Brassica* crop contains an expressible plastidic AHASL gene that encodes a mutation at P197(*At*) or W574(*At*), if that mutation is encoded in a *Brassica* B- or C-genome allele, must also encode at least one additional mutation, in the same or different expressible plastidic AHASL gene, where that mutation is selected from those substitutions at sites: G121(*At*), A122(*At*), M124(*At*), R142(*At*), V196(*At*), R199(*At*), T203(*At*), A205(*At*), F206(*At*), K256(*At*), M351(*At*), H352(*At*), R373(*At*), D375(*At*), D376(*At*), R377(*At*), M570(*At*), V571(*At*), F578(*At*), S653(*At*), and G654(*At*); and preferably at sites from among A122(*At*), R199(*At*), A205(*At*), S653(*At*), and G654(*At*); and more preferably at S653(*At*). Winter-type *Brassica* plants of this type can be employed in the methods described throughout the detailed description hereof, including methods for controlling weeds with performing post-emergent herbicide treatment, methods for selecting plants and methods for providing yield protection.

A method for controlling weeds in a winter-type *Brassica* crop may include the steps of: performing post-emergent treatment of an herbicide-tolerant (HT) *Brassica* plant of said crop by applying an herbicide composition to the plant and its immediate vicinity, at a dose rate in the range from 0.25x to about 4x of SU, wherein said herbicide composition comprises a SU; and said *Brassica* plant (1) comprises at least one herbicide tolerantAHASL (HT-AHASL) gene, wherein one of the HT-AHASL genes encodes a sulfonylurea tolerance HT (SU-HT) mutation selected from P197X and W574X, and at least one additional mutation selected from G121X, A122X, M124X, V196X, R199X,T203X, A205X, F206X, K256X, M351X, H352X, R373X, D375X, D376X, R377X, M570X, V571X, F578X, S653X, and G654X; or (2) comprises at least two herbicide tolerant AHASL (HT-AHASL) genes wherein a first HT-AHASL gene encodes a sulfonylurea tolerance (SU-HT) mutation selected from P197X and W574X and a second HT-AHASL gene encodes a mutation selected from G121X, A122X, M124X, V196X, R199X, T203X, A205X, F206X, K256X, M351X, H352X, R373X, D375X, D376X, R377X, M570X, V571X, F578X, S653X, and G654X.

Said herbicide composition can comprise other agronomically useful forms of sulfonylurea(s). The sulfonylurea may be selected from the group consisting of amidosulfuron, flupyrsulfuron, foramsulfuron, imazosulfuron, iodosulfuron, mesosulfuron, nicosulfuron, thifensulfuron, and tribenuron, agronomically acceptable salts and esters thereof, and combinations thereof. The herbicide composition may comprise a significant amount of no other SU.

Said SU-HT mutation may be selected from P197A, P197S, P197L, and W574L. In some cases, only one of the HT-AHASL genes encodes the SU-HT mutation selected from P197X and W574X. Said at least one additional mutation may be selected from A122T, A122Q, A122V, P197L, P197A, P197S, A205V, R199A, A205V, W574L, S653N, G654E, and G654D. In some cases, said at least one additional mutation is selected from A122T, R199A, A205V, G654E, and S653N.

Having now described the present invention in detail, the same will be more clearly understood by reference to the following examples, which are included herewith for purposes of illustration only.

### EXAMPLES

Specific examples of the preparation of herbicide-tolerant plants which may be employed in methods of the invention are provided below.

As used herein, "no significant" plant injury equates to injuries with a score of 5 or less on the 0-100 PHYTOX scale, described below, preferably injuries having a score of 4, 3, 2, 1, or less. In some embodiments, "no significant" plant injury can be a transient injury lasting 5 days or fewer, and preferably lasting 4, 3, 2, or 1 day or less.

Abbreviations and acronyms used herein are defined as follows:
ED₅₀ = effective dose (i.e., dose required to produce a desired effect in 50% of a population);
DAT = days after treatment;
DALT = days after last treatment;
DAP = days after planting;
GS = growth stage
BBCH = industry-recognized standard for identifying phenological stages of growth in canola

### EXAMPLE 1

Herbicide tolerance in AHAS-inhibitor tolerant WOSR plant lines compared to AHAS-inhibitor susceptible (conventional) WOSR plant lines.

Plants of an AHAS-inhibitor tolerant WOSR line, for example a line a representative seed sample of which is deposited under ATCC Deposit No. 40684, and plants of a first AHAS-inhibitor susceptible WOSR line are sown in 10 cm pots with a sandy loam. Each pot is planted with two plants, the plants are watered from beneath and fertilized according there requirement. The pots are stored side by side in a greenhouse at 12°C at the emergence phase. The temperature increases to 15-20°C three weeks after sowing.

A post-emergence treatment of herbicides is applied to the plants by means of fine distributed nozzles and a water use rate of 200L/ha at the growth stage GS/ BBCH 12 (2 true leaf stage). The herbicides are tested at different rates. Five cultivar replicates are carried out per rate.

The evaluation of efficacy is assessed as crop damage caused by the herbicides using a scale from 0 to 100%, compared to the untreated control plants. Here, 0 means no damage and 100 means complete destruction of the plants. The level of efficacy is assessed 21-22 days after treatment (DAT). The efficacy results are presented as ED₅₀ values.

**TABLE 5**

| | Plants of first AHAS-inhibitor susceptible WOSR line | Plants of AHAS-inhibitor tolerant WOSR line |
|---|---|---|
| AI | ED₅₀ (g/ai/ha) | ED₅₀ (g/ai/ha) |
| Tribenuron-methyl | 2.59 | 25.88 |
| Florasulam | 1.51 | 6.53 |
| Flupyrsulfuron- methy l-sodium | 0.58 | 63316.17 |
| Metsulfuron-methyl | 0.57 | 5.06 |
| Tritosulfuron | 0.39 | 42.30 |
| Sulfosulfuron | 1.70 | 2417.06 |
| Propoxycarbazone-sodium | 2.16 | 67.43 |
| Iodosulfuron Mesosulfuron | 0.27 | 11.31 |
| Chlorsulfuron | 0.42 | 2.8 |
| Trisulfuron-methyl 500 | 3.36 | 41.81 |

### EXAMPLE 2

Herbicide tolerance in AHAS-inhibitor tolerant WOSR plant lines compared to AHAS-inhibitor susceptible (conventional) WOSR plant lines.

Plants of an AHAS-inhibitor tolerant WOSR line and plants of a second AHAS-inhibitor susceptible WOSR line are sown in 10 cm pots with a sandy loam. Each pot is planted with two plants, the plants are watered from beneath and fertilized according there requirement. The pots are stored side by side in a greenhouse at 12°C at the emergence phase. The temperature increases to 15-20°C three weeks after sowing.

A post-emergence treatment of herbicides is applied to the plants by means of fine distributed nozzles and a water use rate of 200L/ha at the growth stage GS/ BBCH 10. The herbicides are tested at different rates. Twelve cultivar replicates are carried out per rate.

The evaluation of efficacy is assessed as crop damage caused by the herbicides using a scale from 0 to 100%, compared to the untreated control plants. Here, 0 means no damage and 100 means complete destruction of the plants. The level of efficacy is assessed 19 days after treatment (DAT). The efficacy results are presented as ED₅₀ values.

**TABLE 6**

| | Plants of second AHAS-inhibitor susceptibleWOSR line | Plants of AHAS-inhibitor tolerantWOSR line |
|---|---|---|
| AI | ED₅₀ (g/ai/ha) | ED₅₀ (g/ai/ha) |
| Tribenuron-methyl | 0.11 | 9.11 |
| Florasulam | 0.28 | 4.9 |
| Flupyrsulfuron-methyl-sodium | 0.79 | 147.97 |
| Metsulfuron-methyl | 0.17 | 2.28 |
| Tritosulfuron | 1.76 | 2.96 |
| Sulfosulfuron | 0.01 | 3.29 |
| Propoxycarbazone-sodium | 0.17 | 42.07 |
| Iodosulfuron Mesosulfuron | 0.07 | 0.62 |
| Chlorsulfuron | 0.04 | 11.2 |
| Trisulfuron-methyl 500 | 0.03 | 4.48 |

### EXAMPLE 3

Enhanced herbicide tolerance in plants of AHAS-tolerant WOSR plant lines compared to plants of AHAS-tolerant spring oilseed rape (SOSR) plant lines.

Plants of an AHAS-inhibitor tolerant WOSR line and plants of an AHAS-tolerant SOSR line are sown side by side in a plot. A post-emergence treatment of herbicides is applied to the plants by means of fine distributed nozzles at the growth stage GS/ BBCH 12/13. There are four replications of each treatment.

The evaluation for crop-tolerance is assessed as PHYTOX symptom caused by the chemical compounds carried out using a scale from 0 to 100%, compared to the untreated control plants. Here, 0 means no damage and 100 means complete destruction of the plants.

**TABLE 7**

| AI | Product Rate (kg/ha) | Crop Variety | WOSR | SOSR |
|---|---|---|---|---|
| | | Crop GS from/to/method | 14/15/B | 13/14/B |
| | | DAT/DALT/DAP | 15/15/37 | 15/15/37 |
| | | Eval. Unit | PHYTOX % | PHYTOX % |
| | | AI Rate (g/ha) | | |
| Flupyrsulfuron-methyl-sodium | 0.01 | 5 | 11 | 19 |
| Thifensulfuron - methyl | 0.03 | 15 | 13 | 23 |
| Tritosulfuron | 0.035 | 25 | 43 | 53 |
| Tribenuron-methyl | 0.03 | 15 | 17 | 25 |

### EXAMPLE 4

Enhanced herbicide tolerance in plants of AHAS-inhibitor tolerant WOSR plant lines compared to plants of AHAS-inhibitor susceptible (conventional) WOSR plant lines.

Plants of an AHAS-inhibitor tolerant WOSR line and plants of an AHAS-inhibitor susceptible WOSR line are sown side by side in a plot. A pre-emergence treatment of herbicides is applied to the plants by means of fine distributed nozzles. There are four replications of each treatment.

The evaluation for crop-tolerance is assessed as PHYTOX symptom caused by the chemical compounds carried out using a scale from 0 to 100%, compared to the untreated control plants. Here, 0 means no damage and 100 means complete destruction of the plants.

**TABLE 8**

| AI | Product Rate (g/ha) | Resistance/Variety | AHAS- inhibitor tolerant WOSR line | AHAS-inhibitor tolerant WOSR line |
|---|---|---|---|---|
| | | Crop GS from/to | 10/11 | 11/13 |
| | | DAT/DALT/DAP | 10/10/10 | 29/29/29 |
| | | Eval. Unit | PHYTOX % | PHYTOX % |
| | | AI Rate (g/ha) | | |
| Propoxycarbazone | 10 | 7 | 0 | 0 |
| Sulfosulfuron | 2.5 | 2 | 0 | 0 |
| Flupyrsulfuron-methyl | 50 | 1.8 | 0 | 0 |
| Amidosulfuron | 20 | 2.75 | 0 | 0 |

**TABLE 9**

| AI | Product Rate (g/ha) | Resistance/Variety | AHAS-inhibitor susceptible WOSR line | AHAS- inhibitor susceptible WOSR line |
|---|---|---|---|---|
| | | Crop GS from/to | 10/11 | 11/13 |
| | | DAT/DALT/DAP | 10/10/10 | 29/29/29 |
| | | Eval. Unit | PHYTOX % | PHYTOX % |
| | | AI Rate (g/ha) | | |
| Propoxycarbazone | 10 | 7 | 100 | 100 |
| Sulfosulfuron | 2.5 | 2 | 99 | 99 |
| Flupyrsulfuron-methyl | 50 | 1.8 | 100 | 100 |
| Amidosulfuron | 20 | 2.75 | 100 | 100 |

### EXAMPLE 5

### AHAS Activity in the Presence of Imidazolinone Herbicides

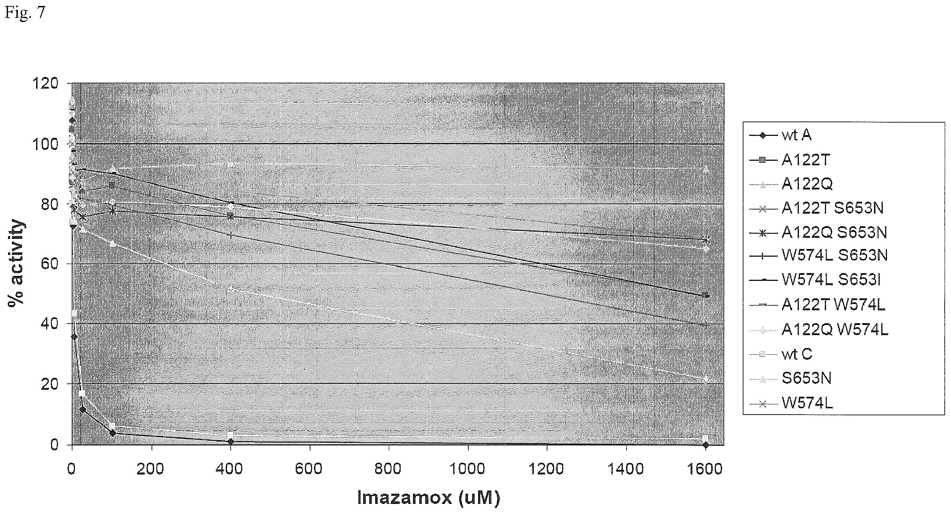

AHAS enzymes with various mutations are reacted with pyruvate and treated with water and serial solutions of varying imazamox concentration to determine AHAS activity. Reactions proceed at 37°C for 45 minutes and are terminated by addition of 20 µL of a solution of 5% sulfuric acid with heating at 60°C for 15-30 minutes to convert acetolactate to acetoin. The resulting acetoin is incubated with creatin and naphthyl (creatin-naphthyl complex) in sodium hydroxide solution at 60°C for 15 minutes to produce a colored product for measurement and correlation with activity of the AHAS enzymes. Fig. 7 shows the AHAS enzyme activity.

### EXAMPLE 6

### AHAS Activity in the Presence of Sulfonylurea Herbicides

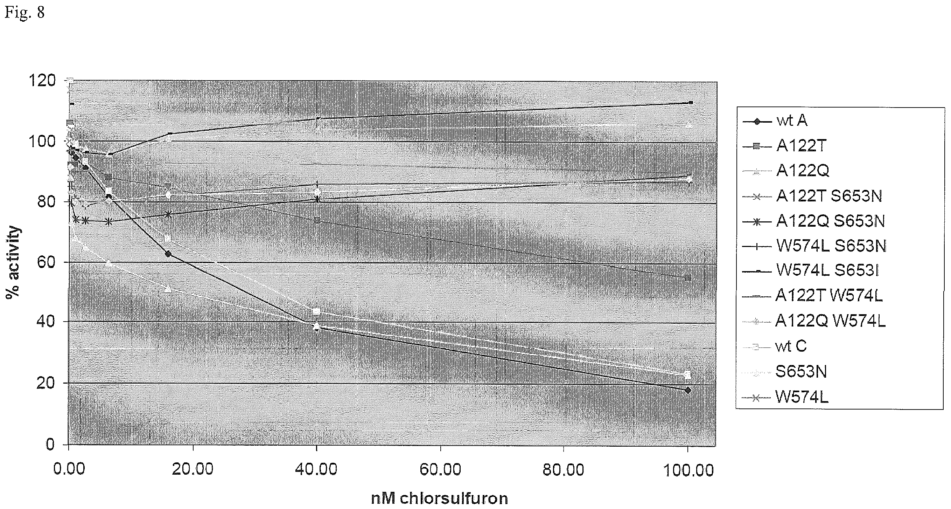

AHAS enzymes with various mutations are reacted with pyruvate and treated with water and serial solutions of varying chlorsulfuron concentration to determine AHAS activity. Reactions proceed at 37°C for 45 minutes and are terminated by addition of 20 µL of a solution of 5% sulfuric acid with heating at 60°C for 15-30 minutes to convert acetolactate to acetoin. The resulting acetoin is incubated with creatin and naphthyl (creatin-naphthyl complex) in sodium hydroxide solution at 60°C for 15 minutes to produce a colored product for measurement and correlation with activity of the AHAS enzymes. Fig. 8 shows the AHAS enzyme activity.

## Claims

1. A method for protecting a winter-type *Brassica* crop from weeds using an herbicide composition comprising sulfonylurea(s) (SU), the method comprising:
growing an herbicide-tolerant (HT) *Brassica* plant of said crop, and
performing treatment of said plant by applying an herbicide composition comprising SU(s) which inhibit the growth of the wild-type version of the *Brassica* plant, wherein
(A) said treatment is:
(1) a post-emergent treatment at a dose rate in the range from 0.25x to 4x of SU, and the herbicide composition:
(a) comprises a SU selected from amidosulfuron, flupyrsulfuron, foramsulfuron, imazosulfuron, iodosulfuron, mesosulfuron, nicosulfuron, thifensulfuron, and tribenuron, agronomically acceptable salts and esters thereof, and combinations thereof, and
(b) optionally comprises not more than 10 wt.% of total Other SU content; or
(2) a pre-emergent treatment, or 0 to 30 days-pre-planting treatment, of the seed planting locus thereof and its immediate vicinity, at a dose rate in the range from greater than 0.5x to 4x of SU and the herbicide composition:
(a) comprises a SU selected from amidosulfuron, flupyrsulfuron, foramsulfuron, imazosulfuron, iodosulfuron, mesosulfuron, nicosulfuron, thifensulfuron, and tribenuron, agronomically acceptable salts and esters thereof, and combinations thereof, and
(b) comprises not more than 3 wt.% of any Other SU; and
(B) said *Brassica* plant comprises at least one herbicide tolerant AHASL (HT-AHASL) gene, wherein only one of the HT-AHASL genes in the plant encodes a sulfonylurea herbicide tolerance (SU-HT) mutation, wherein the SU-HT mutation is W574X, and wherein the HT-AHASL gene encoding the SU-HT mutation is located in the A genome of said *Brassica* plant; and wherein said *Brassica* plant is capable of tolerating said SU treatment at a dose rate of 1x of SU with no significant SU-herbicide-induced injury from said treatment.

2. A method of providing yield protection for a winter-type *Brassica* crop grown in the presence of a sulfonylurea (SU) herbicide composition comprising:
planting a seed of a winter-type *Brassica* plant in the presence of the SU herbicide composition, said herbicide composition comprising (1) a SU selected from amidosulfuron, flupyrsulfuron, foramsulfuron, imazosulfuron, iodosulfuron, mesosulfuron, nicosulfuron, thifensulfuron, and tribenuron, agronomically acceptable salts and esters thereof, and combinations thereof, and (2) optionally not more than 10 wt.% of Other SU content, not more than 5 wt.% of Other SU content, or not more than 3 wt.% of Other SU content; and
growing the seed under conditions capable of producing the winter-type *Brassica* plant;
wherein said *Brassica* plant comprises at least one herbicide tolerant AHASL (HT-AHASL) gene, wherein only one of the HT-AHASL genes in the plant encodes a sulfonylurea herbicide tolerance (SU-HT) mutation, wherein the SU-HT mutation is W574X, and wherein the HT-AHASL gene encoding the SU-HT mutation is located in the A genome of said *Brassica* plant; and
wherein the yield is equal to or greater than the yield provided by a wild-type version of the same type of winter-type *Brassica* plant.

3. A method of providing yield protection for a winter-type *Brassica* crop comprising:
planting a seed of a winter-type *Brassica* plant;
growing the seed under conditions capable of producing the winter-type *Brassica* plant; and
performing an herbicide treatment of the plant by applying an herbicide composition, comprising sulfonylurea(s) (SU), to the plant and its immediate vicinity, said herbicide composition comprising (1) a SU selected from amidosulfuron, flupyrsulfuron, foramsulfuron, imazosulfuron, iodosulfuron, mesosulfuron, nicosulfuron, thifensulfuron, and tribenuron, agronomically acceptable salts and esters thereof, and combinations thereof, and (2) optionally not more than 10 wt.% of Other SU content, not more than 5 wt.% of Other SU content, or not more than 3 wt.% of Other SU content;
wherein said *Brassica* plant comprises at least one herbicide tolerant AHASL (HT-AHASL) gene, wherein only one of the HT-AHASL genes in the plant encodes a sulfonylurea herbicide tolerance (SU-HT) mutation, wherein the SU-HT mutation is W574X, and wherein the HT-AHASL gene encoding the SU-HT mutation is located in the A genome of said *Brassica* plant; and
wherein the yield is equal to or greater than the yield provided by a wild-type version of the same type of winter-type *Brassica* plant.

4. The method of any one of claims 1 to 3 further comprising choosing, prior to treatment with the herbicide composition, said herbicide tolerant *Brassica* plant.

5. The method of any one of claims 1 to 3 further comprising choosing seed capable of producing said herbicide tolerant *Brassica* plant, planting said seed and growing said herbicide tolerant *Brassica* plant from said seed.

6. The method of any one of claims 1 to 3 wherein the HT-AHASL gene encoding the W574X mutation:
(a) encodes W574X homozygously or hemizygously;
(b) encodes W574X in one allele and encodes a wild-type W574W residue in the homologous allele; or
(c) encodes two different W574X mutations heterozygously.

7. A method for selecting an HT winter-type *Brassica* plant comprising:
(I) performing the post-emergent treatment according to items (A)(1) and (B) of claim 1, or performing the pre-emergent treatment, or the 0 to 30 days-pre-planting treatment, according to items (A)(2) and (B) of claim 1; and
(II) selecting a *Brassica* plant capable of tolerating said SU treatment at a dose rate of at least 1x of SU with no significant SU-herbicide-induced injury from said treatment.

8. The method according to any one of claims 1 to 7, wherein said herbicide composition further comprises one or more imidazolinone herbicides.

9. The method according to claim 8, wherein the imidazolinone is imazamox or an agronomically acceptable salt or ester thereof.

10. The method of any one of claims 1 to 9 wherein the SU-HT mutation is selected from W574L, W574M, W574C, W574S, W574R, W574G, W574A, W574F, W574Q, and W574Y.

11. The method of claim 10 wherein the SU-HT mutation is W574L.

12. The method according to any one of claims 1 to 11, wherein the *Brassica* plant further comprises at least one other HT-mutation encoded by at least one HT-AHASL gene of the plant, wherein said other HT-mutation is selected from A122X, R199X, A205X, S653X, G654X, and combinations thereof.

13. The method according to claim 12, wherein the at least one other HT-mutation is selected from A122T, A122V, A122D, A122P, A122Y, R199A, R199E, A205V, A205C, A205D, A205E, A205R, A205T, A205W, A205Y, A205N, S653N, S653I, S653F, S653T, G654Q, G654C, G654E, G654D, and combinations thereof.

14. The method according to any one of claims 2 to 3 wherein weeds present in the immediate vicinity of said winter-type *Brassica* plant are controlled by said SU herbicide composition.

15. The method according to claim 2 wherein the SU herbicide composition is present as a soil residue of an SU herbicide composition applied to a previous crop.

16. The method according to any one of claims 2 to 3 wherein the yield of the winter-type *Brassica* crop is higher than a yield of a crop of winter-type *Brassica* plants of the corresponding wild-type isoline and grown in the same herbicide-containing conditions.

17. The method according to any one of claims 2 to 3 wherein the yield of the winter-type *Brassica* crop is substantially equivalent to the yield when the winter-type Brassica crop is grown in absence of the SU herbicide composition.

18. The method of claim 4 wherein the chosen *Brassica* plant is capable of tolerating said herbicide treatment at a dose rate of at least 1x of SU with no significant SU-herbicide-induced injury from said treatment.

19. The method according to any one of claims 1 to 18, wherein said herbicide composition comprises at least one further active ingredient chosen from among: EPSPS-inhibitors, glyphosate; glutamine synthetase inhibitors, glufosinate; ACCase inhibitors, dims, fops, or dens; PPO-inhibitors, saflufenacil; fungicides, pyraclostrobin; or agronomically acceptable salts or esters thereof; and the *Brassica* plant expresses a trait of tolerance to said further active ingredients.

20. The method of claim 1, wherein the herbicide composition of (A)(1) comprises not more than 5 wt.% of total Other SU content.

21. The method of claim 20, wherein the herbicide composition of (A)(1) comprises not more than 3 wt.% of total Other SU content.

## Patentansprüche

1. Verfahren zum Schützen einer *Brassica*-Kultur des Wintertyps gegen Unkräuter unter Verwendung einer Herbizidzusammensetzung umfassend Sulfonylharnstoff(e) (SH), wobei das Verfahren Folgendes umfasst:
Heranziehen einer herbizidtoleranten (HT) *Brassica*-Pflanze dieser Kultur und
Durchführen einer Behandlung der Pflanze durch Ausbringen einer Herbizidzusammensetzung umfassend (einen) SH(e), der/die das Wachstum der Wildtypversion der *Brassica-*Pflanze hemmt/en, wobei
(A) es sich bei der Behandlung um Folgendes handelt:
(1) eine Nachauflaufbehandlung in einer Aufwandmenge im Bereich von 0,25x bis 4x SH, und wobei die Herbizidzusammensetzung:
(a) einen SH, ausgewählt aus Amidosulfuron, Flupyrsulfuron, Foramsulfuron, Imazosulfuron, Iodosulfuron, Mesosulfuron, Nicosulfuron, Thifensulfuron und Tribenuron, agronomisch annehmbare Salze und Ester davon sowie Kombinationen davon umfasst, und
(b) gegebenenfalls einen Gesamtgehalt an anderen SH von nicht mehr als 10 Gew.-% umfasst; oder
(2) eine Vorauflaufbehandlung, oder eine Behandlung 0 bis 30 Tage vor dem Pflanzen, des Orts des Samenanbaus und seiner unmittelbaren Umgebung mit einer Aufwandmenge im Bereich von mehr als 0,5x bis 4x SH und wobei die Herbizidzusammensetzung:
(a) einen SH ausgewählt aus Amidosulfuron, Flupyrsulfuron, Foramsulfuron, Imazosulfuron, Iodosulfuron, Mesosulfuron, Nicosulfuron, Thifensulfuron und Tribenuron, agronomisch annehmbaren Salzen und Estern davon sowie Kombinationen davon umfasst, und
(b) nicht mehr als 3 Gew.-% eines sonstigen SH umfasst; und
(B) die *Brassica*-Pflanze mindestens ein herbizidtolerantes AHASL(HT-AHASL)-Gen umfasst, wobei nur eines der HT-AHASL-Gene in der Pflanze für eine Sulfonylharnstoffherbizidtoleranz(SU-HT)-Mutation codiert, wobei es sich bei der SU-HT-Mutation um W574X handelt, und wobei sich das HT-AHASL-Gen, das für die SU-HT-Mutation codiert, im A-Genom der *Brassica*-Pflanze befindet; und wobei die *Brassica-*Pflanze fähig ist, die SH-Behandlung mit einer Aufwandmenge von 1x SH zu tolerieren, ohne dass signifikante SH-Herbizid-induzierte Schäden durch die Behandlung entstehen.

2. Verfahren zur Bereitstellung von Ertragsschutz für eine in Gegenwart einer Sulfonylharnstoff(SH)-Herbizidzusammensetzung herangezogenen *Brassica*-Kultur des Wintertyps, das Folgendes umfasst:
Pflanzen eines Samens einer *Brassica*-Pflanze des Wintertyps in Gegenwart der SH-Herbizidzusammensetzung, wobei die Herbizidzusammensetzung (1) einen SH ausgewählt aus Amidosulfuron, Flupyrsulfuron, Foramsulfuron, Imazosulfuron, Iodosulfuron, Mesosulfuron, Nicosulfuron, Thifensulfuron und Tribenuron, agronomisch annehmbaren Salzen und Estern davon sowie Kombinationen davon umfasst, und (2) gegebenenfalls einen Gehalt an anderen SH von nicht mehr als 10 Gew.-%, einen Gehalt an anderen SH von nicht mehr als 5 Gew.-% oder einen Gehalt an anderen SH von nicht mehr als 3 Gew.-% umfasst; und
Heranziehen des Samens unter Bedingungen, die es gestatten, die *Brassica*-Pflanze des Wintertyps zu produzieren;
wobei die *Brassica*-Pflanze mindestens ein herbizidtolerantes AHASL(HT-AHASL)-Gen umfasst, wobei nur eines der HT-AHASL-Gene in der Pflanze für eine Sulfonylharnstoffherbizidtoleranz(SU-HT)-Mutation codiert, wobei es sich bei der SU-HT-Mutation um W574X handelt, und wobei sich das HT-AHASL-Gen, das für die SU-HT-Mutation codiert, im A-Genom der *Brassic*a-Pflanze befindet; und
wobei der Ertrag gleich dem Ertrag, der von einer Wildtypversion desselben Typs einer *Brassica*-Pflanze des Wintertyps bereitgestellt wird, oder größer als dieser ist.

3. Verfahren zum Bereitstellen von Ernteschutz für eine *Brassica*-Kultur des Wintertyps, das Folgendes umfasst:
Pflanzen eines Samens einer *Brassica*-Pflanze des Wintertyps;
Heranziehen des Samens unter Bedingungen, die es gestatten, die *Brassica*-Pflanze des Wintertyps zu produzieren; und
Durchführen einer Herbizidbehandlung der Pflanze durch Ausbringen einer Herbizidzusammensetzung, die Sulfonylharnstoff(e) (SH) umfasst, auf die Pflanze und ihre unmittelbare Umgebung, wobei die Herbizidzusammensetzung (1) einen SH ausgewählt aus Amidosulfuron, Flupyrsulfuron, Foramsulfuron, Imazosulfuron, Iodosulfuron, Mesosulfuron, Nicosulfuron, Thifensulfuron und Tribenuron, agronomisch annehmbaren Salzen und Estern davon sowie Kombinationen davon umfasst und (2) gegebenenfalls einen Gehalt an anderen SH von nicht mehr als 10 Gew.-%, einen Gehalt an anderen SH von nicht mehr als 5 Gew.-% oder einen Gehalt an anderen SH von nicht mehr als 3 Gew.-% umfasst;
wobei die *Brassica*-Pflanze mindestens ein herbizidtolerantes AHASL(HT-AHASL)-Gen umfasst, wobei nur eines der HT-AHASL-Gene in der Pflanze für eine Sulfonylharnstoffherbizidtoleranz(SH-HT)-Mutation codiert, wobei es sich bei der SH-HT-Mutation um W574X handelt, und wobei sich das HT-AHASL-Gen, das für die SH-HT-Mutation codiert, im A-Genom der *Brassica*-Pflanze befindet; und
wobei der Ertrag gleich dem Ertrag, der von einer Wildtypversion desselben Typs einer *Brassica*-Pflanze des Wintertyps bereitgestellt wird, oder größer als dieser ist.

4. Verfahren nach einem der Ansprüche 1 bis 3, das weiterhin Folgendes umfasst: Auswählen der herbizidtoleranten *Brassica*-Pflanze vor der Behandlung mit der Herbizidzusammensetzung.

5. Verfahren nach einem der Ansprüche 1 bis 3, das weiterhin Folgendes umfasst: Auswählen von Samen, der fähig ist, die herbizidtolerante *Brassica*-Pflanze zu produzieren, Pflanzen des Samens und Heranziehen der herbizidtoleranten *Brassica*-Pflanze aus dem Samen.

6. Verfahren nach einem der Ansprüche 1 bis 3, wobei das HT-AHASL-Gen, das für die W574X-Mutation codiert,
(a) W574X homozygot oder hemizygot codiert;
(b) in einem Allel für W574X codiert und in dem homologen Allel für einen Wildtyp W574W-Rest codiert; oder
(c) heterozygot für zwei unterschiedliche W574X-Mutationen codiert.

7. Verfahren zum Auswählen einer HT*-Brassica*-Pflanze des Wintertyps, das Folgendes umfasst:
(I) Durchführen der Nachauflaufbehandlung gemäß der Punkte (A)(1) und (B) nach Anspruch 1, oder Durchführen der Vorauflaufbehandlung, oder der Behandlung 0 bis 30 Tage vor dem Pflanzen, gemäß der Punkte (A) (2) und (B) nach Anspruch 1; und
(II) Auswählen einer *Brassica*-Pflanze, die fähig ist, die SH-Behandlung in einer Aufwandmenge von mindestens 1x SH zu tolerieren, ohne dass signifikante SH-Herbizid-induzierte Schäden durch die Behandlung entstehen.

8. Verfahren nach einem der Ansprüche 1 bis 7, wobei die Herbizidzusammensetzung weiterhin ein oder mehrere Imidazolinonherbizide umfasst.

9. Verfahren nach Anspruch 8, wobei es sich bei dem Imidazolinon um Imazamox oder ein agronomisch unbedenkliches Salz oder einen agronomisch unbedenklichen Ester davon handelt.

10. Verfahren nach einem der Ansprüche 1 bis 9, wobei die SH-HT-Mutation aus W574L, W574M, W574C, W574S, W574R, W574G, W574A, W574F, W574Q und W574Y ausgewählt ist.

11. Verfahren nach Anspruch 10, wobei es sich bei der SH-HT-Mutation um W574L handelt.

12. Verfahren nach einem der Ansprüche 1 bis 11, wobei die *Brassica*-Pflanze weiterhin mindestens eine andere HT-Mutation umfasst, die von mindestens einem HT-AHASL-Gen der Pflanze codiert wird, wobei die andere HT-Mutation aus A122X, R199X, A205X, S653X, G654X und Kombinationen davon ausgewählt ist.

13. Verfahren nach Anspruch 12, wobei die mindestens eine andere HT-Mutation aus A122T, A122V, A122D, A122P, A122Y, R199A, R199E, A205V, A205C, A205D, A205E, A205R, A205T, A205W, A205Y, A205N, S653N, S653I, S653F, S653T, G654Q, G654C, G654E, G654D und Kombinationen davon ausgewählt ist.

14. Verfahren nach einem der Ansprüche 2 bis 3, wobei Unkräuter, die in der unmittelbaren Umgebung der *Brassica*-Pflanze des Wintertyps vorhanden sind, von der SH-Herbizidzusammensetzung bekämpft werden.

15. Verfahren nach Anspruch 2, wobei die SH-Herbizidzusammensetzung als Bodenrückstand einer SH-Herbizidzusammensetzung, die auf eine Vorkultur ausgebracht wurde, vorliegt.

16. Verfahren nach einem der Ansprüche 2 bis 3, wobei der Ertrag der *Brassica*-Kultur des Wintertyps höher ist als ein Ertrag einer Kultur von *Brassica*-Pflanzen des Wintertyps der entsprechenden Wildtyp-Isolinie, die unter denselben herbizidhaltigen Bedingungen herangezogen wird.

17. Verfahren nach einem der Ansprüche 2 bis 3, wobei der Ertrag der *Brassica*-Kultur des Wintertyps dem Ertrag, wenn die *Brassica*-Kultur des Wintertyps in Abwesenheit der SH-Herbizidzusammensetzung herangezogen wird, im Wesentlichen äquivalent ist.

18. Verfahren nach Anspruch 4, wobei die ausgewählte Brassica-Pflanze fähig ist, die Herbizidbehandlung mit einer Aufwandmenge von mindestens 1x SH zu tolerieren, ohne dass signifikante SH-Herbizid-induzierte Schäden durch die Behandlung entstehen.

19. Verfahren nach einem der Ansprüche 1 bis 18, wobei die Herbizidzusammensetzung mindestens einen weiteren Wirkstoff ausgewählt unter den Folgenden umfasst: EPSPS-Hemmern, Glyphosat; Glutaminsynthetasehemmern, Glufosinat; ACCase-Hemmern, Dims, Fops oder Dens; PPO-Hemmern, Saflufenacil; Fungiziden, Pyraclostrobin; oder agronomisch annehmbaren Salzen oder Estern davon; und wobei die *Brassica*-Pflanze ein Merkmal der Toleranz gegenüber den weiteren Wirkstoffen exprimiert.

20. Verfahren nach Anspruch 1, wobei die Herbizidzusammensetzung von (A) (1) einen Gesamtgehalt an anderen SH von nicht mehr als 5 Gew.-% umfasst.

21. Verfahren nach Anspruch 20, wobei die Herbizidzusammensetzung von (A) (1) einen Gesamtgehalt an anderen SH von nicht mehr als 3 Gew.-% umfasst.

## Revendications

1. Méthode de protection d'une culture de *Brassica* de type hiver contre des adventices en utilisant une composition herbicide comprenant une ou plusieurs sulfonylurées (SU), la méthode comprenant :
la culture d'une plante de *Brassica* tolérante aux herbicides (HT) de ladite culture, et
la mise en oeuvre d'un traitement de ladite plante par l'application d'une composition herbicide comprenant une ou plusieurs SU qui inhibe la croissance de la version de type sauvage de la plante de *Brassica,* où
(A) ledit traitement est
(1) un traitement en post-levée à un taux de dose dans la plage allant de 0,25x à 4x de SU, et la composition herbicide :
(a) comprend une SU choisie parmi l'amidosulfuron, le flupyrsulfuron, le foramsulfuron, l'imazosulfuron, l'iodosulfuron, le mésosulfuron, le nicosulfuron, le thifensulfuron, et le tribénuron, les sels et esters acceptables sur le plan agronomique de ceux-ci, et des combinaisons de ceux-ci, et
(b) comprend éventuellement au plus 10% en poids d'une teneur totale en autres SU ; ou
(2) un traitement en pré-levée, ou un traitement à 0 à 30 jours pré-semis, du lieu de plantation des semences et de son entourage immédiat, à un taux de dose dans la plage allant de plus de 0,5x à 4x de SU, et la composition herbicide :
(a) comprend une SU choisie parmi l'amidosulfuron, le flupyrsulfuron, le foramsulfuron, l'imazosulfuron, l'iodosulfuron, le mésosulfuron, le nicosulfuron, le thifensulfuron, et le tribénuron, les sels et esters acceptables sur le plan agronomique de ceux-ci, et des combinaisons de ceux-ci, et
(b) comprend au plus 3% en poids de toute autre SU ; et
(B) ladite plante de *Brassica* comprend au moins un gène AHASL de tolérance aux herbicides (HT-AHASL), où seul l'un des gènes HT-AHASL dans la plante code pour une mutation de tolérance aux herbicides de type sulfonylurée (SU-HT), où la mutation SU-HT est W574X, et où le gène HT-AHASL codant pour la mutation SU-HT est situé dans le génome A de ladite plante de *Brassica* ; et où ladite plante de *Brassica* est capable de tolérer ledit traitement par des SU à un taux de dose de 1x de SU sans lésions significatives induites par l'herbicide de type SU résultant dudit traitement.

2. Méthode consistant à fournir une protection du rendement à une culture de *Brassica* de type hiver cultivée en présence d'une composition herbicide de type sulfonylurée (SU), comprenant :
le semis de semences d'une plante de *Brassica* de type hiver en présence de la composition herbicide de type SU, ladite composition herbicide comprenant (1) une SU choisie parmi l'amidosulfuron, le flupyrsulfuron, le foramsulfuron, l'imazosulfuron, l'iodosulfuron, le mésosulfuron, le nicosulfuron, le thifensulfuron, et le tribénuron, les sels et esters acceptables sur le plan agronomique de ceux-ci, et des combinaisons de ceux-ci, et (2) éventuellement au plus 10% en poids d'une teneur en autres SU, au plus 5% en poids d'une teneur en autres SU, ou au plus 3% en poids d'une teneur en autres SU ; et
la culture des semences dans des conditions capables de produire la plante de *Brassica* de type hiver ;
où ladite plante de *Brassica* comprend au moins un gène AHASL de tolérance aux herbicides (HT-AHASL), où seul l'un des gènes HT-AHASL dans la plante code pour une mutation de tolérance aux herbicides de type sulfonylurée (SU-HT), où la mutation SU-HT est W574X, et où le gène HT-AHASL codant pour la mutation SU-HT est situé dans le génome A de ladite plante de *Brassica* ; et
où le rendement est égal ou supérieur au rendement fourni par une version de type sauvage du même type de plante de *Brassica* de type hiver.

3. Méthode consistant à fournir une protection du rendement à une culture de *Brassica* de type hiver, comprenant :
le semis de semences d'une plante de *Brassica* de type hiver ;
la culture des semences dans des conditions capables de produire la plante de *Brassica* de type hiver ; et
la mise en oeuvre d'un traitement herbicide de la plante par l'application d'une composition herbicide, comprenant une ou plusieurs sulfonylurées (SU), à la plante et son entourage immédiat, ladite composition herbicide comprenant (1) une SU choisie parmi l'amidosulfuron, le flupyrsulfuron, le foramsulfuron, l'imazosulfuron, l'iodosulfuron, le mésosulfuron, le nicosulfuron, le thifensulfuron, et le tribénuron, les sels et esters acceptables sur le plan agronomique de ceux-ci, et des combinaisons de ceux-ci, et (2) éventuellement au plus 10% en poids d'une teneur en autres SU, au plus 5% en poids d'une teneur en autres SU, ou au plus 3% en poids d'une teneur en autres SU ;
où ladite plante de *Brassica* comprend au moins un gène AHASL de tolérance aux herbicides (HT-AHASL), où seul l'un des gènes HT-AHASL dans la plante code pour une mutation de tolérance aux herbicides de type sulfonylurée (SU-HT), où la mutation SU-HT est W574X, et où le gène HT-AHASL codant pour la mutation SU-HT est situé dans le génome A de ladite plante de *Brassica* ; et
où le rendement est égal ou supérieur au rendement fourni par une version de type sauvage du même type de plante de *Brassica* de type hiver.

4. Méthode selon l'une quelconque des revendications 1 à 3, comprenant en outre le choix, préalablement au traitement par la composition herbicide, de ladite plante de *Brassica* tolérante aux herbicides.

5. Méthode selon l'une quelconque des revendications 1 à 3, comprenant en outre le choix de semences capables de produire ladite plante de *Brassica* tolérante aux herbicides, le semis desdites semences et la culture de ladite plante de *Brassica* tolérante aux herbicides à partir desdites semences.

6. Méthode selon l'une quelconque des revendications 1 à 3, dans laquelle le gène HT-AHASL codant pour la mutation W574X
(a) code pour W574X de manière homozygote ou hémizygote ;
(b) code pour W574X dans un allèle et code pour un résidu W574W de type sauvage dans l'allèle homologue ; ou
(c) code pour deux mutations W574X différentes de manière hétérozygote.

7. Méthode de sélection d'une plante de *Brassica* de type hiver HT, comprenant :
(I) la mise en oeuvre du traitement en post-levée selon les articles (A) (1) et (B) selon la revendication 1, ou la mise en oeuvre du traitement en pré-levée, ou du traitement à 0 à 30 jours pré-semis selon les articles (A) (2) et (B) selon la revendication 1 ; et
(II) la sélection d'une plante de *Brassica* capable de tolérer ledit traitement par des SU à un taux de dose d'au moins 1x de SU sans lésions significatives induites par l'herbicide de type SU résultant dudit traitement.

8. Méthode selon l'une quelconque des revendications 1 à 7, dans laquelle ladite composition herbicide comprend en outre un ou plusieurs herbicides à base d'imidazolinone.

9. Méthode selon la revendication 8, dans laquelle l'imidazolinone est l'imazamox ou un sel ou ester acceptable sur le plan agronomique de celui-ci.

10. Méthode selon l'une quelconque des revendications 1 à 9, dans laquelle la mutation SU-HT est choisie parmi W574L, W574M, W574C, W574S, W574R, W574G, W574A, W574F, W574Q et W574Y.

11. Méthode selon la revendication 10, dans laquelle la mutation SU-HT est W574L.

12. Méthode selon l'une quelconque des revendications 1 à 11, dans laquelle la plante de *Brassica* comprend en outre au moins une autre mutation HT codée par au moins un gène HT-AHASL de la plante, où ladite autre mutation HT est choisie parmi A122X, R199X, A205X, S653X, G654X, et des combinaisons de celles-ci.

13. Méthode selon la revendication 12, dans laquelle la au moins une autre mutation HT est choisie parmi A122T, A122V, A122D, A122P, A122Y, R199A, R199E, A205V, A205C, A205D, A205E, A205R, A205T, A205W, A205Y, A205N, S653N, S653I, S653F, S653T, G654Q, G654C, G654E, G654D, et des combinaisons de celles-ci.

14. Méthode selon l'une quelconque des revendications 2 à 3, dans laquelle des adventices présentes dans l'entourage immédiat de ladite plante de *Brassica* de type hiver sont contrôlées par ladite composition herbicide de type SU.

15. Méthode selon la revendication 2, dans laquelle la composition herbicide de type SU est présente sous forme d'un résidu au sol d'une composition herbicide de type SU appliquée à une culture précédente.

16. Méthode selon l'une quelconque des revendications 2 à 3, dans laquelle le rendement de la culture de *Brassica* de type hiver est supérieur à un rendement d'une culture de plantes de *Brassica* de type hiver de la lignée isogénique de type sauvage correspondante et cultivée dans les mêmes conditions de contenance en herbicide.

17. Méthode selon l'une quelconque des revendications 2 à 3, dans laquelle le rendement de la culture de *Brassica* de type hiver est sensiblement équivalent au rendement lorsque la culture de *Brassica* de type hiver est cultivée en l'absence de la composition herbicide de type SU.

18. Méthode selon la revendication 4, dans laquelle la plante de *Brassica* choisie est capable de tolérer ledit traitement herbicide à un taux de dose d'au moins 1x de SU sans lésions significatives induites par l'herbicide de type SU résultant dudit traitement.

19. Méthode selon l'une quelconque des revendications 1 à 18, dans laquelle ladite composition herbicide comprend au moins un autre ingrédient actif choisi parmi les inhibiteurs d'EPSPS, le glyphosate ; les inhibiteurs de la glutamine synthétase, le glufosinate ; les inhibiteurs d'ACCase, les herbicides de type DIM, FOP ou DEN ; les inhibiteurs de PPO, le saflufénacil ; les fongicides, la pyraclostrobine ; ou les sels ou esters acceptables sur le plan agronomique de ceux-ci ; et la plante de *Brassica* exprime un caractère de tolérance vis-à-vis desdits autres ingrédients actifs.

20. Méthode selon la revendication 1, dans laquelle la composition herbicide de (A) (1) comprend au plus 5% en poids d'une teneur totale en autres SU.

21. Méthode selon la revendication 20, dans laquelle la composition herbicide de (A) (1) comprend au plus 3% en poids d'une teneur totale en autres SU.
